(19) Europäisches Patentamt
European Patent Office
Office européen des brevets

(11) EP 4 245 751 A1

(12) EUROPEAN PATENT APPLICATION
published in accordance with Art. 153(4) EPC

(43) Date of publication:
**20.09.2023 Bulletin 2023/38**

(21) Application number: **21891815.9**

(22) Date of filing: **08.11.2021**

(51) International Patent Classification (IPC):
***C07C 281/06*** *(2006.01)* ***C09D 175/12*** *(2006.01)*
***C08G 18/32*** *(2006.01)* ***C09D 7/65*** *(2018.01)*

(52) Cooperative Patent Classification (CPC):
**C07C 281/06; C08G 18/32; C09D 7/65; C09D 175/12**

(86) International application number:
**PCT/JP2021/040965**

(87) International publication number:
**WO 2022/102569 (19.05.2022 Gazette 2022/20)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **10.11.2020 JP 2020187273**

(71) Applicant: **Asahi Kasei Kabushiki Kaisha**
**Tokyo 1000006 (JP)**

(72) Inventors:
- **INNAMI Yu**
  **Tokyo 100-0006 (JP)**
- **YAMAUCHI Toyoaki**
  **Tokyo 100-0006 (JP)**

(74) Representative: **Strehl Schübel-Hopf & Partner**
**Maximilianstrasse 54**
**80538 München (DE)**

# (54) SEMICARBAZIDE COMPOSITION AND AQUEOUS COATING COMPOSITION

(57) The present invention provides a semicarbazide composition containing a semicarbazide compound (S) derived from an isocyanate compound (N) and a hydrazine, wherein the semicarbazide compound (S) has a hydrophilic functional group (a) and a structure (b) represented by the following formula (1), and a molar ratio (b)/(a) of the structure (b) to the hydrophilic functional group (a) is 0.1 or more and 10.0 or less, and the like.

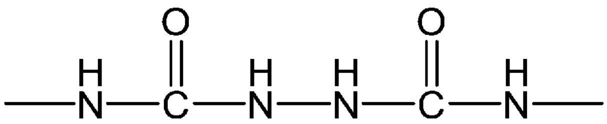

( I )

EP 4 245 751 A1

## Description

[Technical field]

The present invention relates to a semicarbazide composition and a water-based coating composition.

[Background Art]

Water-based coating compositions, which use water as a medium, can reduce volatile organic compounds (VOCs) compared to organic solvent-based coating compositions, and are replacing organic solvent-based coating compositions in accordance with environmental regulations. In recent years, not only environmental regulations but also the safety for users and surroundings have been attracting more attention, and the importance of water-based coating compositions has increased.

However, water-based coating compositions consume more energy than organic solvents when drying the coating compositions, resulting in an increase in $CO_2$ emissions. Therefore, in order to reduce $CO_2$ emissions from water-based coating compositions, attempts have been made to reduce the energy used during drying and curing, that is, to lower the heating temperature. However, when the heating temperature is lowered, the curing reaction between the curing agent and the resin in the water-based coating compositions becomes insufficient, which causes a problem in that the physical properties of the coating film tend to deteriorate.

As a technology for rapid curing even at low of normal temperature, a hydrazone crosslinking utilizing a dehydration condensation reaction between a curing agent obtained from a hydrazine derivative and a resin having a carbonyl group can be mentioned. For example, a water-based coating composition has been proposed in which a semicarbazide compound, which is a reaction product of isocyanate and a hydrazine, is used as a curing agent and subjected to a cross-linking reaction with a carbonyl group-containing resin (see, for example, Patent Documents 1 and 2). Further, Patent Documents 3 to 5 disclose semicarbazide compositions which are free from sediments even in water-based coating compositions, have good storage stability, and provide good water resistance to the coating films obtained.

[Citation List]

[Patent Document]

[Patent Document 1] Japanese Patent No. 3073201
[Patent Document 2] Japanese Patent No. 3521991
[Patent document 3] Japanese Patent No. 5990277
[Patent Document 4] Japanese Unexamined Patent Application, First Publication No. 2005-42023
[Patent Document 5] PCT International Publication WO2020/11100

[Summary of Invention]

[Technical Problem]

However, the semicarbazide compositions disclosed in Patent Documents 1 and 2 use a semicarbazide compound derived from an isocyanate oligomer derived from a diisocyanate monomer and a hydrazine and having a relatively large molecular weight. Therefore, when the semicarbazide compound and the resin having a carbonyl group partially react in the coating composition, the dispersibility or solubility in the water-based coating composition decreases, and sedimentation and gelation occur, resulting in storage stability issues.

A semicarbazide compound having a hydrophilic functional group introduced therein has also been proposed in order to improve the dispersion stability in water-based coating compositions, but there is also the problem in that the hydrophilic functional group reduces the water resistance of the coating film.

Furthermore, the semicarbazide compositions disclosed in Patent Documents 3 to 5, etc. also have the problem in that the elongation of the resulting coating film is reduced due to the semicarbazide compound derived from the alicyclic isocyanate.

As described above, a coating film using a semicarbazide derived from an alicyclic isocyanate has excellent water resistance, but poor elongation, and when an aliphatic isocyanate is introduced to improve the elongation, there is a problem of poor storage stability.

In addition, in order to ensure good storage stability (solubility and dispersibility in water,) without introducing

a hydrophilic group, almost 100% of aliphatic isocyanate or almost 100% of alicyclic isocyanate can be used. However, in this case, it is impossible to achieve both water resistance and elongation.

Therefore, it has been desired to develop a coating composition having both storage stability, and water resistance and elongation when formed into a coating film.

The inventors of the present invention found that a double urea bond (structure (b)) contributes to water resistance, and have developed a composition containing an aliphatic isocyanate that contributes to elongation as a main component. The structure (b) is produced by adding an excess amount of an isocyanate group to the hydrazine, but since the ratio of semicarbazide groups is relatively low, the formation of the structure (b) is conventionally suppressed by adding excess hydrazine (see, for example, Patent Document 3, etc.). None of Patent Documents 1 to 5 describe that the structure (b) improves the water resistance, and do not provide any motivation to make the structure (b) a specific range. Moreover, there is neither description nor suggestion that 50 wt% or more of aliphatic isocyanate is used as the isocyanate compound.

The present invention has been made in view of the above circumstances, and provides a semicarbazide composition that has excellent storage stability when made into a water-based coating composition and excellent water resistance and elongation when made into a coating film, and a water-based coating composition containing the semicarbazide composition.

[Solution to Problem]

That is, the present invention includes the following aspects.

(1) A semicarbazide composition containing a semicarbazide compound (S) derived from an isocyanate compound (N) and a hydrazine, wherein

the semicarbazide compound (S) has a hydrophilic functional group (a) and a structure (b) represented by the following formula (1), and

a molar ratio (b)/(a) of the structure (b) to the hydrophilic functional group (a) is 0.1 or more and 10.0 or less.

[Chemical formula 1]

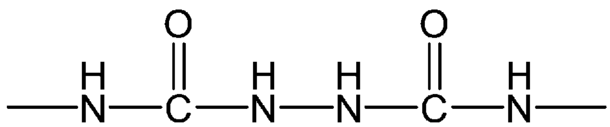

(I)

(2) The semicarbazide composition according to (1), wherein the semicarbazide compound (S) includes a semicarbazide compound derived from a hydrazine and at least one isocyanate compound (N) selected from the group consisting of an aliphatic isocyanate and an alicyclic isocyanate.

(3) The semicarbazide composition according to (1) or (2), wherein

the semicarbazide compound (S) includes a semicarbazide compound derived from an aliphatic isocyanate and a hydrazine, and

a content of the aliphatic isocyanate is 10% by mass or more and 99% by mass or less with respect to a total mass of the isocyanate compound (N).

(4) The semicarbazide composition according to any one of (1) to (3), wherein

the isocyanate compound (N) includes an aliphatic isocyanate and an alicyclic isocyanate, and

a content of the aliphatic isocyanate is 10% by mass or more and 99% by mass or less with respect to a total mass of the isocyanate compound (N).

(5) The semicarbazide composition according to any one of (1) to (4), wherein the isocyanate compound (N) includes at least one compound selected from the group consisting of a butane diisocyanate, a pentamethylene diisocyanate, a hexamethylene diisocyanate, a trimethylhexamethylene diisocyanate, a 4-isocyanatomethyl-1,8-octamethylene diisocyanate, a lysine ester triisocyanate and a polyisocyanate derived therefrom.

(6) The semicarbazide composition according to any one of (1) to (5), wherein the hydrophilic functional group (a) is one or more functional groups selected from the group consisting of a nonionic hydrophilic functional group and

an anionic hydrophilic functional group.

(7) The semicarbazide composition according to any one of (1) to (6), wherein a content of the hydrophilic functional group (a) is 5% by mass or more and 40% by mass or less with respect to a total mass of the semicarbazide compound (S).

(8) The semicarbazide composition according to any one of (1) to (7), further comprising a hydrophilic organic solvent (H).

(9) The semicarbazide composition according to (8), wherein a content of the hydrophilic organic solvent (H) is 0.5% by mass or more and 50% by mass or less with respect to a total mass of the semicarbazide composition.

(10) A water-based coating composition, comprising the semicarbazide composition according to any one of (1) to (9), and a resin (C) having a carbonyl group.

[Advantageous Effects of Invention]

According to the semicarbazide composition of the above aspect, it is possible to provide a semicarbazide composition which has excellent storage stability when made into a water-based coating composition and excellent water resistance and elongation when made into a coating film. The water-based coating composition of the above aspect contains the semicarbazide composition and has excellent storage stability, water resistance and elongation when formed into a coating film.

[Description of Embodiments]

Hereinafter, the embodiments (hereinafter, referred to as "the present embodiment") for implementing the present invention are demonstrated in detail. The following embodiments are examples explaining the present invention, and are not intended to limit the present invention to the following contents. The present invention can be appropriately modified and implemented within the scope of the present invention.

<<Semicarbazide composition>>

The semicarbazide composition of this embodiment contains a semicarbazide compound (S) derived from an isocyanate compound (N) and hydrazine. The semicarbazide compound (S) includes a hydrophilic functional group (a) and a structure (b) represented by the following formula (1) (hereinafter sometimes simply referred to as "structure (b)"). The structure (b) has a structure in which two urea bonds are linked.

[Chemical formula 2]

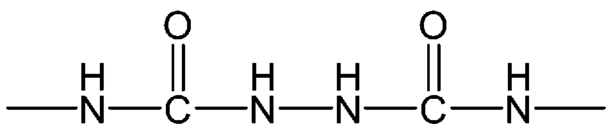

(I)

In the semicarbazide composition of the present embodiment, the molar ratio (b)/(a) of the structure (b) to the hydrophilic functional group (a) is 0.1 or more and 10.0 or less, preferably 0.5 or more and 9.5 or less, and more preferably 0.9 or more and 9.0 or less.

When the molar ratio (b)/(a) is at least the above lower limit, the water resistance of the coating film can be improved. On the other hand, when it is equal to or less than the above upper limit, an excessive cohesive force of the compound can be suppressed, the dispersibility and solubility of the coating composition can be improved, and the storage stability can be improved.

The molar ratio (b)/(a) can be calculated, for example, by measuring the molar amount of the hydrophilic functional group (a) and the molar amount of the structure (b) in the semicarbazide composition by $^{13}C$-NMR measurement. Specific measurement conditions are as shown in the Examples described later.

Since the semicarbazide composition of the present embodiment has the above structure, it has excellent storage stability when made into a water-based coating composition and excellent water resistance and elongation when made into a coating film.

Next, each component contained in the semicarbazide composition of the present embodiment will be described in detail below.

<Semicarbazide compound (S)>

In general, a "semicarbazide compound" is a compound derived from an isocyanate compound and hydrazine, i.e., a reaction product of an isocyanate compound and hydrazine.

In the semicarbazide composition of the present embodiment, the semicarbazide compound (S) may be an isomer thereof. In addition, the semicarbazide compound (S) may have at least one functional group at the terminal of the molecule which is a semicarbazide group, and may have a functional group other than the semicarbazide group in addition to the semicarbazide group.

The semicarbazide compound (S) contains a hydrophilic functional group (a) and a structure (b) represented by formula (I) below.

[Chemical formula 3]

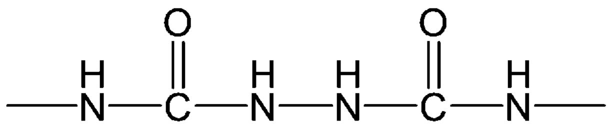

(I)

[Hydrophilic functional group (a)]

The semicarbazide compound (S) contains a hydrophilic functional group (a). By including the hydrophilic functional group (a), it is possible to improve the dispersibility and solubility of the coating composition, and also possible to improve the storage stability. The hydrophilic functional group (a) herein does not include semicarbazide group.

The hydrophilic functional group (a) is preferably one or more functional groups selected from the group consisting of nonionic hydrophilic functional groups and anionic hydrophilic functional groups. By selecting these functional groups, compatibility with anionic resins and additives generally used for water-based coating compositions is better.

Examples of the nonionic hydrophilic functional groups include polyoxyalkylene groups, polyoxyalkylenearyl groups, sorbitan derivatives, glycerin ester derivatives and the like. Among them, a polyoxyalkylene group is preferable because of its excellent dispersion stability.

Examples of the anionic hydrophilic functional groups include sulfonic acid groups, carboxylic acid groups, phosphoric acid groups and the like.

Moreover, as the hydrophilic compound from which the hydrophilic functional group (a) is derived, a compound having a functional group that reacts with a semicarbazide group and the hydrophilic functional group (a) is preferable. Examples of such compounds include compounds having a carbonyl group such as a diacetone alcohol, pyruvic acid, acetoacetic acid, levulinic acid, succinic anhydride or the like. Among them, a levulinic acid is preferable because the functional group after the reaction with the semicarbazide group is easily stabilized in water.

Alternatively, as the hydrophilic compound from which the hydrophilic functional group (a) is derived, a compound having a functional group that reacts with an isocyanate compound and the hydrophilic functional group (a) is preferable. Examples of the functional groups that react with the isocyanate compounds include hydroxyl groups, mercapto groups, carboxylic acid groups, amino groups, and thiol groups. Examples of such hydrophilic compounds include, but are not particularly limited to, anionic compounds, nonionic compounds and the like. These hydrophilic compounds may be used singly or in combination of two or more.

(Anionic compound)

Although the anionic compound is not particularly limited, examples thereof include a compound having a carboxylic acid group, a compound having a phosphoric acid group, a compound having a sulfonic acid group, and the like.

Although the compounds having a carboxylic acid group are not particularly limited, examples thereof include carboxylic acids having a hydroxyl group such as monohydroxycarboxylic acids such as 1-hydroxyacetic acid, 3-hydroxypropanoic acid, 12-hydroxy-9-octadecanoic acid, hydroxypivalic acid, lactic acid or the like; polyhydroxycarboxylic acids such as dimethylolacetic acid, 2,2-dimethylolbutyric acid, 2,2-dimethylolpentanoic acid, dihydroxysuccinic acid and dimethylolpropionic acid or the like; or the like. Among them, hydroxypivalic acid or dimethylolpropionic acid is preferred.

Although the compounds having a phosphate group are not particularly limited, examples thereof include an acid phosphate, acid phosphite, acid hypophosphite, certain polyether phosphonates (e.g., those commercially available under the trade name RHODAFAC® (manufactured by Solvay Nicca, Ltd.)). Among them, acidic phosphates are preferable.

Although the compounds having a sulfonic acid group are not particularly limited, examples thereof include a

sulfonic acid having a hydroxyl group, a sulfonic acid having an amino group, and the like. Examples of the sulfonic acids having a hydroxyl group include 2-hydroxyethanesulfonic acid, 3-hydroxypropanesulfonic acid, 4-hydroxybutanesulfonic acid, 5-hydroxypentanesulfonic acid, 6-hydroxyhexanesulfonic acid, hydroxybenzenesulfonic acid, hydroxy(methyl)benzenesulfonic acid, 4-(2-hydroxyethyl)-1-piperazineethanesulfonic acid, 4-(2-hydroxyethyl)-1-piperazinepropanesulfonic acid, 2-hydroxy-3-morpholinopropanesulfonic acid, certain polyether sulfonates (e.g., those commercially available under the trade name Tegomer® (manufactured by The Goldschmidt AG, Essen, Germany)). Examples of the sulfonic acids having an amino group include 2-aminoethanesulfonic acid, 2-methylaminoethanesulfonic acid, 2-(cyclohexylamino)-ethanesulfonic acid, 3-(cyclohexylamino)-propanesulfonic acid, 4-aminotoluene-2-sulfonic acid, 5-aminotoluene-2-sulfonic acid, 2-aminonaphthalene-4-sulfonic acid, 4-aminobenzenesulfonic acid, 3-aminobenzenesulfonic acid and the like.

Among them, 2-hydroxyethanesulfonic acid, 3-hydroxypropanesulfonic acid, hydroxybenzenesulfonic acid, hydroxy(methyl)benzenesulfonic acid, 2-(cyclohexylamino)-ethanesulfonic acid, and 3-(cyclohexylamino)-propanesulfone acids are preferable.

The acidic groups such as carboxylic acid groups, phosphoric acid groups and sulfonic acid groups of the anionic compounds are preferably neutralized with an inorganic base or an organic amine compound.

Examples of the inorganic base include alkali metals such as lithium, sodium, potassium, rubidium, cesium or the like; alkaline earth metals such as magnesium, calcium, strontium, barium or the like; metals such as manganese, iron, cobalt, nickel, copper, zinc, silver, cadmium, lead, aluminum or the like; and ammonia.

Examples of the organic amine compound include linear tertiary amines such as trimethylamine, triethylamine, tripropylamine, tributylamine, trioctylamine, trilaurylamine, tridecylamine, tristearylamine or the like; branched tertiary amines such as triisopropylamine, triisobutylamine, tri-2-ethylhexylamine, tri-branched tridecylamine or the like; tertiary amines having mixed hydrocarbon groups such as N,N-dimethylethylamine, N,N-dimethylpropylamine, N,N-dimethylisopropylamine, N,N-dimethylbutylamine, N,N-dimethylisobutylamine, N,N-dimethyloctylamine, N,N- dimethyl-2-ethylhexylamine, N,N-dimethyllaurylamine, N,N-dimethyl (branched) tridecylamine, N,N-dimethylstearylamine, N,N-diethylbutylamine, N,N-diethylhexylamine, N,N-diethyloctylamine, N,N-diethyl-2-ethylhexylamine, N,N-diethyllaurylamine, N,N-diisopropylmethylamine, N,N-diisopropylethylamine, N,N-diisopropylbutylamine, N,N-diisopropyl-2-ethylhexylamine or the like; alicyclic tertiary amines such as N,N-dimethylcyclohexylamine, N,N-diethylbenzylamine, N,N-diethylcyclohexylamine, N,N-dicyclohexylmethylamine, N,N-dicyclohexylethylamine, tricyclohexylamine or the like; tertiary amines having an aromatic ring substituent such as N,N-dimethylbenzylamine, N,N-diethylbenzylamine, N,N-dibenzylmethylamine, tribenzylamine, N,N-dimethyl-4-methylbenzylamine, N,N-dimethylphenylamine, N , N-diethylphenylamine, N,N-diphenylmethylamine or the like; cyclic amines such as N-methylpyrrolidine, N-ethylpyrrolidine, N-propylpyrrolidine, N-butylpyrrolidine, N-methylpiperidine, N-ethylpiperidine, N-propylpiperidine, N-butylpiperidine, N-methylmorpholine, N-ethylmorpholine, N-propylmorpholine, N-butylmorpholine, N-sec-butylmorpholine, N-tert-butylmorpholine, N-isobutylmorpholine, quinuclidine or the like; and the like. These organic amine compounds may be used singly or in combination of two or more.

Among them, tertiary amines having 5 to 30 carbon atoms are preferable, and specific examples include triethylamine, tripropylamine, tributylamine, trioctylamine, trilaurylamine, tridecylamine, triisopropylamine, triisobutylamine, tri-2-ethylhexylamine, tri-branched tridecylamine, N,N-dimethylpropylamine, N,N-dimethylisopropylamine, N,N-dimethylbutylamine, N,N-dimethylisobutylamine, N,N-dimethyloctylamine, N,N-dimethyl-2-ethylhexylamine, N,N-dimethyllaurylamine, N,N-dimethyl (branched) tridecylamine, N,N-dimethylstearylamine, N,N-diethylbutylamine, N,N-diethylhexylamine, N,N-diethyloctylamine, N,N-diethyl-2-ethylhexylamine, N,N-diethyllaurylamine, N,N-diisopropylmethylamine, N,N-diisopropylethylamine, N,N-dimethylcyclohexylamine, N,N-diethylcyclohexylamine, N,N-dicyclohexylmethylamine, N,N-dicyclohexylethylamine, N,N-dimethylbenzylamine, N,N-diethylbenzylamine, N,N-dibenzylmethylamine, tribenzylamine, N,N-dimethylphenylamine, N,N-diethylphenylamine, N,N-diphenylmethylamine, N-methylpiperidine, N-ethylpiperidine, N-methylmorpholine, N-ethylmorpholine, quinuclidine, pyridine, quinoline and the like. These preferred organic amine compounds may be used singly or in combination of two or more.

The semicarbazide compound is modified with a hydrophilic compound (introducing the hydrophilic functional group (a) derived from a hydrophilic compound into the semicarbazide compound (S)) in order to disperse it in water. By not increasing the modification ratio too much, there is a tendency to be able to suppress the deterioration of the coating film physical properties (hardness, water resistance and solvent resistance). That is, since the anionic compound has high emulsifying power, a high emulsifying effect can be obtained with a small amount.

Although the method for reacting the raw material isocyanate compound with the anionic compound is not limited to the following, examples thereof include a method of reacting a terminal isocyanate group of a raw material isocyanate compound with an active hydrogen group of an anionic compound.

(Nonionic compound)

Although the nonionic compounds are not particularly limited, examples thereof include polyalkylene glycol

alkyl ethers. From the viewpoint of reducing the viscosity of the polyisocyanate composition, the number of hydroxyl groups possessed by the polyalkylene glycol alkyl ether is preferably 1. The polyalkylene glycol alkyl ether preferably has a structure represented by the following general formula (II).

[Chemical formula 4]

$$HO-\left(R^{21}O\right)_{n21}-R^{22} \qquad (II)$$

(in the general formula (11), $R^{21}$ is an alkylene group having 1 to 4 carbon atoms, $R^{22}$ is an alkyl group having 1 to 4 carbon atoms, and n21 is 4.0 to 20.)

The polyalkylene glycol alkyl ether is not a single component but an aggregate of substances with different numbers of n21 indicating the polymerization degree (hereinafter sometimes referred to as "polymerization degree n21" or simply "n21"). Therefore, the polymerization degree n21 is represented by its average value.

When blending a polyisocyanate into a water-based main agent, thickening when mixed with the main agent often poses a problem. If the viscosity is excessively increased, the polyisocyanate cannot be uniformly dispersed in the main agent, which tends to lead to deterioration of the physical properties of the coating film.

Therefore, n21 is 4.0 or more and 20 or less, preferably 4.0 or more and 16 or less, and more preferably 4.0 or more and 12 or less, from the viewpoint of water dispersibility and dispersibility in the main agent. When n21 is at least the above lower limit, since the emulsifying power increases, the dispersibility tends to improve. On the other hand, when n21 is equal to or less than the above upper limit, since the viscosity can be prevented from increasing, the viscosity tends to be improved.

As the polyalkylene glycol alkyl ether, two or more of those with different n21 are used in combination. n21 of the polyalkylene glycol alkyl ether can be measured by a proton nuclear magnetic resonance (NMR) method.

In the general formula (11), $R^{21}$ is preferably an alkylene group having 1 to 4 carbon atoms from the viewpoint of imparting hydrophilicity, and is preferably an ethylene group having 2 carbon atoms from the viewpoint of imparting greater hydrophilicity.

$R^{22}$ is preferably an alkyl group having 1 or more and 4 or less carbon atoms from the viewpoint of imparting hydrophilicity, and is preferably a methyl group having 1 carbon atom from the viewpoint of imparting more hydrophilicity.

Specific examples of the polyalkylene glycol alkyl ether include, but are not limited to, a polyethylene glycol (mono) methyl ether, poly (ethylene, propylene) glycol (mono) methyl ether, and a polyethylene glycol (mono) ethyl ether. Among them, a polyethylene glycol (mono) methyl ether is preferable from the viewpoint of imparting hydrophilicity.

The content of the hydrophilic functional group (a) is preferably 5% by mass or more and 40% by mass or less, more preferably 7% by mass or more and 39% by mass or less, even more preferably 9% by mass or more and 37% by mass or less, with respect to the total mass of the semicarbazide compound (S). When the content of the hydrophilic functional group (a) is at least the above lower limit, storage stability of the coating composition is further improved. In addition, when the content of the hydrophilic functional group (a) is equal to or less than the above upper limit, water resistance of the coating film is further improved.

The content of the hydrophilic functional group (a) can be determined, for example, using a method of calculating from the content of the hydrophilic functional group (a) in each raw material used for production, of using a method of calculating from the component ratio of the hydrophilic functional group (a) derived from each raw material contained in the composition by analyzing the obtained semicarbazide composition by pyrolysis gas chromatography, NMR, or the like.

[Structure (b)]

Structure (b) consists of a structure in which two urea bonds are linked, and is a structure obtained by reacting a semicarbazide group and an isocyanate group. The structure (b) is believed to have very strong hydrogen bonding and exhibit high cohesion. Therefore, a coating film obtained using a compound containing the structure (b) is less likely to absorb water around the structure (b), and an improvement in water resistance can be expected.

Furthermore, a coating film obtained using a compound containing the structure (b) is preferable because it can achieve both elongation and water resistance of the coating film. Until now, methods to improve the water resistance of the coating film generally include improving the hydrophobicity of the coating film, reducing the hydrophilic component, increasing the hardness of the coating film to make it difficult to absorb water, and increasing the cross-linking density to form a dense coating film. Therefore, the elongation of the coating film with improved water resistance tends to be poor, and it has been difficult to achieve both water resistance and elongation.

On the other hand, even if the elongation of the coating film is increased, the coating film having the structure

(b) does not easily absorb water due to the presumed mechanism described above, and it is possible to achieve both water resistance and elongation. More specifically, a coating film obtained from a semicarbazide compound (S) having the structure (b) in the semicarbazide compound (S) and containing a large amount of an aliphatic isocyanate with high elongation among the isocyanate compounds (N) described later is preferable because it has excellent water resistance and elongation.

The content of structure (b) in the semicarbazide composition of the present embodiment can be controlled, for example, by adjusting the blending amount of the isocyanate compound to be reacted with the compound containing a semicarbazide group, or the molar ratio of hydrazine groups to isocyanate groups when reacting hydrazine with an isocyanate compound.

[Isocyanate compound (N)]

The isocyanate compound (N) used for producing the semicarbazide compound (S) may be any known compound having one or more isocyanate groups in the molecule, and compounds derived from isocyanate compound monomers, such as polyisocyanates having an isocyanurate ring, a biuret bond, a uretdione bond, an allophanate bond, a urethane bond, a urea bond or the like, can also be used.

Preferred isocyanate compounds (N) include, for example, one or more isocyanate compounds selected from the group consisting of aliphatic isocyanates and alicyclic isocyanates. By including one or more isocyanate compounds selected from the group consisting of aliphatic isocyanates and alicyclic isocyanates in the isocyanate compound (N) used in the production of the semicarbazide compound (S), the weather resistance of the resulting coating film can be improved.

Among them, it is more preferable that the isocyanate compound (N) include an aliphatic isocyanate. The content of the aliphatic isocyanate is preferably 10% by mass or more and 99% by mass or less, more preferably 15% by mass or more and 95% by mass or less, even more preferably 20% by mass or more and 90% by mass or less, with respect to the total mass of the isocyanate compound (N). When the content of the aliphatic isocyanate is at least the above lower limit, the elongation of the coating film using the semicarbazide composition is further improved. Further, when the content of the aliphatic isocyanate is equal to or less than the above upper limit, the hardness and glass transition temperature Tg of the coating film using the semicarbazide composition are further improved, and the water resistance is also further improved.

The content of the aliphatic isocyanate can be calculated, for example, by a method of calculating from the blending amount of the aliphatic isocyanate in the isocyanate compound (N) at the time of production of the semicarbazide compound (S), a method of calculating the ratio of the peak derived from the aliphatic isocyanate from the peak derived from the isocyanate compound (N) detected by pyrolysis gas chromatography using semicarbazide composition, or the like.

Furthermore, the isocyanate compound (N) includes an aliphatic isocyanate and an alicyclic isocyanate, and the content of the aliphatic isocyanate is more preferably within the above numerical range. Including an alicyclic isocyanate in addition to the aliphatic isocyanate further improves the water resistance of the coating film using the semicarbazide composition.

Examples of the aliphatic isocyanates include a butane diisocyanate, hexamethylene diisocyanate (HDI), pentamethylene diisocyanate (PDI), trimethylhexamethylene diisocyanate, 4-isocyanatomethyl-1,8-octamethylene diisocyanate (TTI), lysine ester triisocyanate (LTI), polyisocyanates derived from these aliphatic isocyanates, and the like. Among these aliphatic isocyanates, HDI, PDI, LTI, TTI, or polyisocyanates derived therefrom are preferable. Moreover, the polyisocyanate preferably includes an isocyanurate ring, a biuret bond, a uretdione bond, an allophanate bond, a urethane bond and the like, and more preferably includes a biuret bond, an allophanate bond or a urethane bond. A semicarbazide composition using the polyisocyanate having these bonds has improved dispersibility, solubility or the like when used as a coating composition.

Examples of the alicyclic isocyanates include an isophorone diisocyanate (IPDI), 1,3-bis(isocyanatomethyl)-cyclohexane (hydrogenated XDI), 4,4'-dicyclohexylmethane diisocyanate (hydrogenated MDI), polyisocyanates derived from these alicyclic isocyanates, and the like.

These isocyanate compounds may be used singly or in combination of two or more.

Further, the isocyanate compound (N) may have some of the isocyanate groups protected with a blocking agent, or may be modified with an alcohol compound, an amine compound, or the like.

[Hydrazine]

Examples of the hydrazines used for producing the semicarbazide compound (S) include a hydrazine ($NH_2NH_2$); monoalkyl-substituted hydrazine compounds such as a monomethylhydrazine, monoethylhydrazine, monobutylhydrazine or the like; an ethylene-1,2-dihydrazine, a propylene-1 ,3-dihydrazine, a butylene-1 ,4-dihydrazine and the like.

Among them, hydrazine is preferable from the viewpoint of having excellent reactivity between the produced semicarbazide group and the carbonyl group of the resin. Although both anhydride and monohydrate hydrazine can be used, it is preferable to use hydrazine monohydrate ($NH_2NH_2 \cdot H_2O$) from the viewpoint of production safety.

[Method for producing semicarbazide compound (S)]

The semicarbazide compound (S) can be produced using known techniques.

The semicarbazide compound (S) can be obtained by, for example, producing a compound having a semicarbazide group, followed by simultaneously or sequentially reacting the compound having a semicarbazide group with a hydrophilic compound and a compound having an isocyanate group to obtain a semicarbazide compound (S) having a hydrophilic functional group (a) and a structure (b).

Alternatively, a hydrophilic functional group (a) is reacted with a modified isocyanate compound in which a hydrophilic functional group is introduced into some of the isocyanate groups by reacting with a hydrophilic compound in advance to obtain a semicarbazide compound (S) having a hydrophilic functional group (a) and a structure (b). Examples of the hydrophilic compound and the compound having an isocyanate group include those exemplified above for the hydrophilic functional group (a) and the isocyanate compound (N), respectively.

The compound having a semicarbazide group can be produced by the methods described in, for example, Japanese Patent No. 3073201 (Patent Document 1) and Japanese Patent No. 5990277 (Patent Document 3).

Examples of the method for producing the semicarbazide compound having a hydrophilic functional group (a) include a method in which an isocyanate compound is reacted with a hydrophilic compound to introduce a hydrophilic functional group into the isocyanate groups (the isocyanate compound is modified with a hydrophilic compound), and then the resulting modified isocyanate compound is reacted with hydrazine to produce a semicarbazide compound, a method of introducing a hydrophilic functional group into a semicarbazide compound by reacting a semicarbazide compound with a compound containing a functional group that reacts with the semicarbazide group and a hydrophilic functional group, and the like.

<Hydrophilic organic solvent (H)>

The semicarbazide composition of the present embodiment can further contain a hydrophilic organic solvent (H). The term "hydrophilic organic solvent" as used herein refers to an organic solvent having a solubility in water of 10 g/L or more.

The semicarbazide composition of the present embodiment has a strong interaction between semicarbazide molecules in water, such as structures with a strong hydrogen bonding force such as semicarbazide groups or the structure (b), structures derived from isocyanate compounds having a hydrophobic interaction, or the like. Therefore, the advantages of including the hydrophilic organic solvent (H) are that the solubility and dispersibility of the coating composition are further improved, the storage stability is further improved, and the viscosity of the composition is reduced and workability is further improved.

Although the hydrophilic organic solvent (H) may not be included depending on the structure of the isocyanate compound (N) and the amount of the hydrophilic functional group (b), the content of the hydrophilic organic solvent (H) is preferably 0.5% by mass or more and 50% by mass or less, more preferably 1.0% by mass or more and 45% by mass or less, even more preferably 2% by mass or more and 40% by mass or less, with respect to the total mass of the semicarbazide composition. When the content of the hydrophilic organic solvent (H) is at least the above lower limit, the dispersibility and solubility of the semicarbazide composition when used as a coating composition are further improved. On the other hand, when the content of the hydrophilic organic solvent (H) is equal to or less than the above upper limit, the dispersion stability of the resin and the like in the water-based coating composition containing the semicarbazide composition is not impaired.

Examples of the hydrophilic organic solvents (H) include methanol, ethanol, isopropanol, 2-butanol, butyl cellosolve (ethylene glycol monobutyl ether), butyl carbitol, propylene glycol monomethyl ether, diethylene glycol dimethyl ether, dipropylene glycol dimethyl ether, ethylene glycol, dimethyl sulfoxide and the like.

<<Water-based coating composition composition>>

The water-based coating composition of the present embodiment contains the above semicarbazide composition and a resin (C) having a carbonyl group (hereinafter sometimes referred to as "resin (C)").

Each component contained in the water-based coating composition of the present embodiment will be described below.

<Resin (C) having carbonyl group>

Resin (C) is a water-soluble or water-dispersible resin having two or more carbonyl groups in one molecular skeleton. The "carbonyl group" contained in the resin (C) as used herein refers to a functional group that contributes to the cross-linking reaction with the semicarbazide compound (S), and examples thereof include an aldehyde group, a keto group, an anhydride carboxyl group and the like.

Examples of the resin (C) include conventionally known polycarbonyl compounds such as a polyurethane polymer, polyester polymer, poly(meth)acrylate polymer, polyvinyl acetate polymer, polybutadiene-based polymer, polyvinyl chloride polymer, chlorinated polypropylene polymer, polyethylene polymer, fluoropolymer, polystyrene polymer, polystyrene-(meth)acrylate copolymer, rosin derivative, styrene-maleic anhydride copolymer and an alcohol adduct thereof, cellulose resin, and the like. These polycarbonyl compounds may be used singly or in combination of two or more.

Also, the resin (C) can be obtained by copolymerizing or addition-polymerizing a polymerizable monomer having at least one carbonyl group in the molecule with another polymerizable monomer.

Specific examples of the polymerizable monomer having at least one carbonyl group in the molecule include an acetone dicarboxylic acid, dihydroxyacetone, monohydroxyacetone, dihydroxybenzaldehyde, etc., carboxylic anhydride, etc., ethylenically unsaturated monomer etc. having at least one aldehyde group or keto group in the molecule. The resin (C) can be obtained by addition polymerization of these monomers singly or in combination of two or more.

Further, specific examples of the ethylenically unsaturated monomers having at least one carbonyl group in the molecule include compounds containing an aldehyde group or keto group, such as an acrolein, diacetone acrylamide, diacetone methacrylamide, formyl styrene, vinyl methyl ketone, vinyl ethyl ketone, vinyl isobutyl ketone, acryloxyalkylpropanals, methacryloxyalkylpropanals, diacetone acrylate, diacetone methacrylate, acetonyl acrylate, 2-hydroxypropyl acrylate acetylacetate, butanediol-1,4-acrylate acetylacetate, anhydrous succinate, and the like. By copolymerizing an ethylenically unsaturated monomer mixture containing one or more of these polymerizable monomers and an ethylenically unsaturated monomer other than these, the resin (C), that is, a polycarbonyl compound can be obtained.

Furthermore, the resin (C) may contain a functional group that undergoes a cross-linking reaction with another functional group in addition to the bond that undergoes a cross-linking reaction with the semicarbazide group, or may form a cross-link. Examples of the crosslinked structures formed by such a crosslink reaction include a siloxane crosslink by silanol condensation, a urethane crosslink by a hydroxyl group and isocyanate group, a crosslink by a hydroxyl group and melamine, an amide ester crosslink by oxazoline and a carboxyl group, an acyl urea crosslink by a carboxyl group and a carbodiimide group, a cross-link by a carboxy group or by an amino group and an epoxy group, and the like.

Specific examples of the monomer having a crosslinkable functional group include γ-(meth)acryloxypropyltrimethoxysilane, vinylmethyldiethoxysilane, vinylmethyldimethoxysilane, vinyldimethylethoxysilane, vinyldimethylmethoxysilane, vinyltrimethoxysilane, vinyltriethoxysilane, (meth) 2-hydroxyethyl acrylate, (meth) 2-hydroxypropyl acrylate, (meth) ethylene glycol acrylate, ethylene glycol methoxy(meth)acrylate, diethylene glycol (meth)acrylate, diethylene glycol methoxy(meth)acrylate, (meth) tetraethylene glycol acrylate, tetraethylene glycol methoxy(meth)acrylate, (poly)oxypropylene (meth)acrylate, (meth) propylene glycol acrylate, propylene glycol methoxy(meth)acrylate, dipropylene glycol (meth)acrylate, dipropylene glycol methoxy(meth)acrylate, (meth)acrylate tetrapropylene glycol, tetrapropylene glycol methoxy(meth)acrylate, 2-hydroxycyclohexyl (meth)acrylate, acrylic acid, methacrylic acid, itaconic acid, (meth)glycidyl acrylate, allyl glycidyl ether, glycidyl cinnamate, glycidyl crotonate, glycidyl itaconate, glycidyl norbornenyl ester, glycidyl norbornenyl ether, and the like. These polymerizable monomers may be used singly or in combination of two or more.

<Other components>

In addition to the above semicarbazide composition and the above resin (C), the water-based coating composition of the present embodiment may contain other components such as curing agents having functional groups other than semicarbazide groups, synthetic resins and emulsion particles that crosslink with them, a defoamer, colorant, thickener, thixotropic agent, cryostat, matting agent, cross-linking reaction catalyst, pigment, curing catalyst, crosslinker, filler, anti-skinning agent, dispersant, wetting agent, light stabilizer, antioxidant, UV absorber, rheology control agent, defoamer, deposition aid, anti-rust, dye, plasticizer, lubricant, reducing agent, preservative, antifungal agent, deodorant, anti-yellowing agent, antistatic agent or a charge control agent within a range that does not impair the effects thereof.

Examples of the curing agents having functional groups other than semicarbazide groups and isocyanate groups include a carbodiimide, oxazoline, silane coupling agent and the like.

<Method for producing water-based coating composition>

For example, the water-based coating composition can be obtained by adding the semicarbazide composition, the resin (C), and, if necessary, other components, and mixing them by stirring or the like. Further, the water-based

coating composition of the present embodiment can be adjusted to a desired solid content by further adding a hydrophilic organic solvent and water. Examples of the hydrophilic organic solvent include those exemplified in the above "semicarbazide composition".

<Usage>

The water-based coating composition of the present embodiment is suitable, for example, as a curing agent for building exterior coatings, interior materials, automotive coatings, adhesives, inks, binders for electronic materials, or the like.

[EXAMPLSES]

The present invention will be specifically described using the following Production Examples, Examples, and Comparative Examples, but these are not intended to limit the scope of the present invention. The weighed values of raw materials used in the following Production Examples, Examples, and Comparative Examples are expressed in parts by mass unless otherwise specified.

<Method for measuring physical properties>

The physical properties of the isocyanate compound (N), the semicarbazide composition, and the carbonyl group-containing resin (C) were measured by the following measuring methods.

[Physical property 1]

(NCO content)

The NCO content (% by mass) of each isocyanate compound was calculated using the following method.

First, about 1 g or more and 3 g or less of an isocyanate compound was accurately weighed (Wg). Then, 20 mL of toluene was added to the isocyanate compound and dissolved. Next, 10 mL of a 2.0 N di-n-butylamine toluene solution was added to the toluene solution containing the isocyanate compound, and after mixing, the mixture was allowed to stand at room temperature for 15 minutes. Then, 70 mL of isopropyl alcohol was added to this mixture and mixed. This liquid was titrated with a 1.0 N hydrochloric acid solution, and the obtained titration value was defined as "V2 mL". The same operation was performed without the isocyanate compound, and the obtained titration value was defined as "V1 mL". These titration values were used to calculate the NCO group content of the isocyanate compound from the following formula.

$$\text{NCO group content (\% by mass)} = (V1\text{-}V2) \times 42 / (W \times 1000) \times 100$$

[Physical property 2]

(Solid content)

The solid content (% by mass) of each semicarbazide composition and each resin was calculated using the following method.

First, each semicarbazide composition and each resin was used as a sample, and the mass of an aluminum cup was accurately weighed (W0 g). About 1 g of the sample was placed and the mass of the cup before heat drying was accurately weighed (W1 g). The cup containing the above sample was heated in a dryer at 105°C for 1 hour. After cooling the heated cup to room temperature, the mass of the cup was accurately weighed again (W2 g). The accurately weighed masses were used to calculate the mass% of the dry residue in the sample as the solid content (% by mass) from the following formula.

$$\text{Solid content (\% by mass)} = (W2\text{-}W0) / (W1\text{-}W0) \times 100$$

[Physical property 3]

(Content of hydrophilic functional group (a))

The content of the hydrophilic functional group (a) was calculated by the following method.

First, the content of the hydrophilic functional group (a1) contained in the isocyanate compound (N) obtained in each synthesis example was calculated. Specifically, the mass (w1 g) of the isocyanate compound used in the synthesis example and the mass (w2 g) of the raw material containing the hydrophilic functional group were used to calculate the content of the hydrophilic functional group (a1) (mass %) from the following formula.

$$\text{Content of hydrophilic functional group (a1) (\% by mass)} = w2 / (w1 + w2) \times 100$$

Next, the mass (w3 g) of the isocyanate compound (N) used in the production of the semicarbazide composition, the mass (w4 g) of the solid content of the hydrazine or semicarbazide composition used for reacting with the isocyanate compound (N), and the mass (w5g) of the compound having a hydrophilic functional group (a2) other than the isocyanate compound (N) were used to calculate the content (% by mass) of the hydrophilic functional group (a) contained in the semicarbazide compound (S) from the following formula.

$$\text{Content of hydrophilic functional group (a) (\% by mass)} = (w3 \times \text{Content of hydrophilic functional group (a1)} / 100 + w5) / (w3 + w4 + w5) \times 100$$

[Physical property 4]

(Content of aliphatic isocyanate (% by mass))

The content (% by mass) of the aliphatic isocyanate was determined by the following method.

The mass of the aliphatic isocyanate compound (w1 g) used in the production of the semicarbazide composition, the mass of the alicyclic isocyanate (w2 g), the mass of the isocyanate compound contained in the semicarbazide composition used for reacting with the isocyanate compound (w3 g)), and the content ratio (R% by mass) of the aliphatic isocyanate compound among the isocyanate compounds contained in the semicarbazide composition were used to calculate the content (% by mass) of the aliphatic isocyanate from the following formula.

$$\text{Aliphatic isocyanate content (\% by mass)} = (w1 + w3 \times R / 100) / (w1 + w2 + w3) \times 100$$

[Physical property 5]

(Molar ratio of structure (b) to hydrophilic functional group (a) (b)/(a))

The molar ratio (b)/(a) of the structure (b) to the hydrophilic functional group (a) contained in each semicarbazide composition was calculated using the following method.

First, 0.5 g of each semicarbazide composition and 0.1 g of heavy DMSO were weighed and mixed until uniform, then the mixed solution was transferred to an NMR tube and sealed. Then, $^{13}C$-NMR measurement was performed using JEOL-ECZ500 manufactured by JEOL to determine the peak area value $C_b$ of carbon derived from the structure (b) and the peak area value $C_a$ of carbon derived from the hydrophilic functional group (a), and the molar ratio (b)/(a) was calculated from the following formula.

$$\text{Molar ratio (b)/(a)} = C_b / C_a$$

<Evaluation method>

The water-based coating compositions obtained in Examples and Comparative Examples and the coating films

using them were evaluated by the following evaluation methods.

[Evaluation 1]

(Storage stability)

Each water-based coating composition was transferred to a glass bottle, sealed, and allowed to stand still for 10 days in a 40°C atmosphere. After 10 days, the mixture was cooled to room temperature, the coating composition was filtered through a 200-mesh filter cloth, the residue remaining on the filter cloth was visually observed, and the storage stability was evaluated according to the following evaluation criteria.

(Evaluation criteria)

◎: Almost no residue was observed
o: A very small amount of residue was observed
△: Some residue was observed
×: A large amount of residue was observed

[Evaluation 2]

(Water resistance of coating film)

Each water-based coating composition was applied to a mild steel plate coated with a black cationic electrodeposition coating with an applicator so as to give a resin film thickness of 40 μm. After coating, it was pre-dried for 5 minutes at a temperature of 23°C and a humidity of 50%, then placed in an oven at 80°C and dried for 30 minutes to form a coating film, and then cooled to room temperature to prepare a test piece. The obtained test piece was immersed in a plastic cup filled with ion-exchange water, covered with a plastic wrap so that the water did not volatilize, and allowed to stand at 40°C for 10 days. After 10 days, the test piece was taken out, water droplets on the surface were removed, the state of the coating film was visually observed, and the water resistance was evaluated according to the following evaluation criteria.

(Evaluation criteria)

◎: No change was observed in the coating film
o: The coating film was slightly whitened
△: Slight blistering occurred or the coating film was subtly whitened
×: The coating film was markedly whitened

[Evaluation 3]

(Elongation of coating film)

Each water-based coating composition was applied onto a polypropylene plate (PP plate) with an applicator so that the resin film thickness was 40 μm. The coated PP plate was pre-dried at a temperature of 23°C and a humidity of 50% for 5 minutes, then placed in an oven at 80°C and dried for 30 minutes to obtain a coating film. After cooling the obtained coating film to room temperature, a piece of the coating film having a width of 1 cm and a length of 4 cm was cut out from the PP plate. Next, a tensile test was performed using the coated film piece at a speed of 20 mm/min under an atmosphere of 23°C and 50% humidity. The elongation from the start of the test to the breakage of the coating film was determined. Five test pieces were measured, and the average elongation until breakage was taken as the elongation (%) of the coating film. When the elongation was 100% or more, the elongation of the coating film was evaluated as good.

<Synthesis of isocyanate compound (N)>

[Synthesis Example 1-1]

(Synthesis of isocyanate compound N-1)

A four-necked flask equipped with a stirrer, thermometer, reflux condenser, nitrogen blowing tube, and a dropping funnel was placed in a nitrogen atmosphere. In the flask, 750 g of HDI and 8.0 g of water as a biuretizing agent were dissolved in 250 g of a 1:1 (mass ratio) mixed solvent of ethylene glycol methyl ether acetate and trimethyl phosphate, and reacted at a reaction temperature of 160°C for 1 hour. After filtering the obtained reaction solution, unreacted HDI was removed by a thin film distillation apparatus to obtain a polyisocyanate having a biuret skeleton. The NCO group content of the obtained polyisocyanate was 23.5% by mass.

Then, 130.7 g of the obtained polyisocyanate, 76.0 g of polyethylene glycol monomethyl ether (manufactured by Nippon Nyukazai Co., Ltd., trade name "MPG-081", number-average molecular weight: 690), and 0.02 g of 2-ethylhexyl acid phosphate (manufactured by Johoku Chemical Co., Ltd., trade name "JP-508T") were weighed into a flask and reacted in a nitrogen atmosphere with stirring at 90°C for 8 hours. After completion of the reaction, the mixture was cooled to room temperature to obtain isocyanate compound N-1. The resulting isocyanate compound N-1 had an NCO group content of 12.0% by mass, a hydrophilic functional group (a) content of 36.8% by mass, and an aliphatic isocyanate content with respect to the total mass of the isocyanate raw material of 100% by mass.

[Synthesis Example 1-2]

<Production of isocyanate compound N-2>

A four-necked flask equipped with a stirrer, thermometer, reflux condenser, nitrogen blowing tube, and a dropping funnel was placed in a nitrogen atmosphere. In the flask, 750 g of HDI and 8.0 g of water as a biuretizing agent were dissolved in 250 g of a 1:1 (mass ratio) mixed solvent of ethylene glycol methyl ether acetate and trimethyl phosphate, and reacted at a reaction temperature of 160°C for 1 hour. After filtering the obtained reaction solution, unreacted HDI was removed by a thin film distillation apparatus to obtain a polyisocyanate having a biuret skeleton. The NCO group content of the obtained polyisocyanate was 23.5% by mass.

Then, 146.1 g of the obtained polyisocyanate, 56.6 g of MPG-081, and 0.02 g JP-508T were weighed into a flask and reacted in a nitrogen atmosphere with stirring at 90°C for 7 hours. After completion of the reaction, the mixture was cooled to room temperature to obtain isocyanate compound N-2. The resulting isocyanate compound N-2 had an NCO group content of 14.9% by mass, a hydrophilic functional group (a) content of 27.9% by mass, and an aliphatic isocyanate content with respect to the total mass of the isocyanate raw material of 100% by mass.

[Synthesis Example 1-3]

<Production of isocyanate compound N-3>

A four-necked flask equipped with a stirrer, thermometer, reflux condenser, nitrogen blowing tube, and a dropping funnel was placed in a nitrogen atmosphere. In the flask, 750 g of HDI and 8.0 g of water as a biuretizing agent were dissolved in 250 g of a 1:1 (mass ratio) mixed solvent of ethylene glycol methyl ether acetate and trimethyl phosphate, and reacted at a reaction temperature of 160°C for 1 hour. After filtering the obtained reaction solution, unreacted HDI was removed by a thin film distillation apparatus to obtain a polyisocyanate having a biuret skeleton. The NCO group content of the obtained polyisocyanate was 23.5% by mass.

Then, 130.7 g of the obtained polyisocyanate, 101.3 g of MPG-081, and 0.02 g JP-508T were weighed into a flask and reacted in a nitrogen atmosphere with stirring at 90°C for 9 hours. After completion of the reaction, the mixture was cooled to room temperature to obtain isocyanate compound N-3. The resulting isocyanate compound N-3 had an NCO group content of 10.0% by mass, a hydrophilic functional group (a) content of 43.7% by mass, and an aliphatic isocyanate content with respect to the total mass of the isocyanate raw material of 100% by mass.

[Synthesis Example 1-4]

<Production of isocyanate compound N-4>

The inside of a four-necked flask equipped with a stirrer, thermometer, and a condenser tube was replaced with nitrogen, 1,000 g of HDI was charged, and 0.1 g of tetramethylammonium capriate as a catalyst and 1.9 g of

isobutanol were simultaneously added while stirring at 70°C. After 4 hours, it was confirmed by refractive index measurement of the reaction solution that the conversion rate of HDI to isocyanurate was 24% by mass, and 0.2 g of phosphoric acid was added to stop the reaction. Thereafter, after filtering the reaction solution, unreacted HDI was removed by a thin film distillation apparatus to obtain a polyisocyanate having an isocyanurate skeleton. The NCO group content of the obtained polyisocyanate was 23.3% by mass.

Then, 130.7 g of the obtained polyisocyanate, 75.3 g of MPG-081, and 0.02 g JP-508T were weighed into a flask and reacted in a nitrogen atmosphere with stirring at 120°C for 4 hours. After completion of the reaction, the mixture was cooled to room temperature to obtain isocyanate compound N-4. The resulting isocyanate compound N-4 had an NCO group content of 12.3% by mass, a hydrophilic functional group (a) content of 36.5% by mass, and an aliphatic isocyanate content with respect to the total mass of the isocyanate raw material of 100% by mass.

[Synthesis Example 1-5]

<Production of isocyanate compound N-5>

A four-necked flask equipped with a stirrer, thermometer, reflux condenser, nitrogen blowing tube, and a dropping funnel was placed in a nitrogen atmosphere, and 733.3 g of HDI and 54.4 g of polycaprolactone-based polyester polyol (manufactured by Daicel Chemical Industries, Ltd., trade name "PLAXEL 303", molecular weight of 300), which is a trihydric alcohol, were charged. The resulting mixture was subjected to a urethanization reaction while stirring and maintaining the temperature inside the reactor at 90°C for 1 hour. Thereafter, the temperature in the reactor was maintained at 80°C, tetramethylammonium capriate was added as an isocyanurate catalyst, and when the yield reached 50% by mass, phosphoric acid was added to stop the reaction. Then, the reaction solution was raised to 90°C while stirring, and the temperature was maintained for 1 hour, then cooled to room temperature, and the reaction solution was filtered. Thereafter, unreacted HDI was removed using a thin film evaporator to obtain a polyisocyanate having an isocyanurate skeleton. The NCO group content of the obtained polyisocyanate was 18.4% by mass.

Then, 130.7 g of the obtained polyisocyanate, 99.1 g of MPG-081, and 0.02 g JP-508T were weighed into a flask and reacted in a nitrogen atmosphere with stirring at 120°C for 5 hours. After completion of the reaction, the mixture was cooled to room temperature to obtain isocyanate compound N-5. The resulting isocyanate compound N-5 had an NCO group content of 7.4% by mass, a hydrophilic functional group (a) content of 43.1 % by mass, and an aliphatic isocyanate content with respect to the total mass of the isocyanate raw material of 100% by mass.

[Synthesis Example 1-6]

<Production of isocyanate compound N-6>

A four-necked flask equipped with a stirrer, thermometer, reflux condenser, nitrogen blowing tube, and a dropping funnel was placed in a nitrogen atmosphere, and 1,000 g of HDI and 30 g of 2-ethyl-1-hexanol were charged. The resulting mixture was subjected to a urethanization reaction at 80°C for 2 hours while stirring to complete the urethanization. Then, 0.03 g of tetramethylammonium capryate was added as an isocyanurate catalyst at 80°C. After 4 hours, 0.12 g of phosphoric acid was added to stop the reaction when the conversion rate to polyisocyanate reached 28% by mass by measuring the isocyanate group content and refractive index of the reaction solution. Subsequently, unreacted HDI was removed using a thin film evaporator to obtain a polyisocyanate having an allophanate skeleton and an isocyanurate skeleton. The NCO group content of the obtained polyisocyanate was 20.7% by mass.

Then, 130.7 g of the obtained polyisocyanate, 44.5 g of MPG-081, and 0.02 g JP-508T were weighed into a flask and reacted in a nitrogen atmosphere with stirring at 120°C for 3 hours. After completion of the reaction, the mixture was cooled to room temperature to obtain isocyanate compound N-6. The resulting isocyanate compound N-6 had an NCO group content of 13.6% by mass, a hydrophilic functional group (a) content of 25.4% by mass, and an aliphatic isocyanate content with respect to the total mass of the isocyanate raw material of 100% by mass.

[Synthesis Example 1-7]

<Production of isocyanate compound N-7>

A four-necked flask equipped with a stirrer, thermometer, reflux condenser, nitrogen blowing tube, and a dropping funnel was placed in a nitrogen atmosphere, and 1,000 g of HDI and 337 g of polycaprolactone triol having a number-average molecular weight of 850 were charged. The resulting mixture was subjected to a urethanization reaction while stirring and maintaining the temperature inside the reactor at 95°C for 90 minutes. After filtering the cooled reaction solution, unreacted HDI was removed using a thin film evaporator to obtain a polyisocyanate having an adduct skeleton.

The NCO group content of the obtained polyisocyanate was 9.0% by mass.

Then, 130.7 g of the obtained polyisocyanate, 19.4 g of MPG-081, and 0.02 g JP-508T were weighed into a flask and reacted in a nitrogen atmosphere with stirring at 120°C for 3 hours. After completion of the reaction, the mixture was cooled to room temperature to obtain isocyanate compound N-7. The resulting isocyanate compound N-7 had an NCO group content of 6.7% by mass, a hydrophilic functional group (a) content of 12.9% by mass, and an aliphatic isocyanate content with respect to the total mass of the isocyanate raw material of 100% by mass.

<Synthesis of semicarbazide composition having no hydrophilic functional group>

[Synthesis Example 2-1]

(Synthesis of semicarbazide composition S-c1)

128.8 g of ion-exchange water and 42.7 g of hydrazine monohydrate were introduced into a 2L-reactor equipped with a reflux condenser, a dropping tank, a thermometer and a stirrer device while nitrogen was introduced at a rate of 1 L/min. The mixture was stirred while maintaining the temperature in the reactor at 10°C. Thereafter, a mixture of 381 g of cyclohexane and 100 g of isophorone diisocyanate (hereinafter abbreviated as "IPDI") was added dropwise over 3 hours while stirring, and the mixture was stirred for 30 minutes while maintaining the temperature at 10°C. Then, a mixture of 14.4 g of cyclohexane and 3.8 g of HDI was added dropwise over 10 minutes, and the mixture was stirred for 20 minutes after completion of the dropwise addition. After that, the obtained solution was transferred to a separating funnel, and after standing overnight, the aqueous solution of the lower phase was removed from the separated two phases to obtain a semicarbazide composition S-c1 having a solid content of 50% by mass. The semicarbazide composition S-c1 did not contain a hydrophilic functional group (a), and had an aliphatic isocyanate content of 3.7% by mass relative to the total mass of isocyanate raw materials.

<Synthesis of resin (C) having carbonyl group>

[Synthesis Example 3-1]

(Production of resin C-1 having carbonyl group)

514.5 g of ion-exchange water and 12.0 g of an aqueous solution containing 30% by mass of surfactant (trade name: Newcol707SF, manufactured by Nippon Nyukazai Co., Ltd., anionic surfactant, non-volatile content of 30% by mass) were charged into a reactor equipped with a reflux condenser, a dropping tank, a thermometer and a stirrer. Then, after raising the temperature in the reactor to 80°C, a mixed solution of 12.6 g of methacrylic acid, 5.4 g of acrylic acid, 442.8 g of butyl acrylate, 54.0 g of diacetone acrylamide, 315 g of methyl methacrylate, 70.2 g of butyl methacrylate, 0.9 g of dodecyl mercaptan, 477 g of ion-exchange water, 30 g of Newcol 707SF and 1.2 g of ammonium persulfate was allowed to flow into the reactor from the dropping tank over 3 hours. The temperature in the reactor was kept at 80°C during the inflow. After completion of the inflow, the temperature in the reactor was raised to 80°C and maintained for 1 hour and 30 minutes. After that, the reaction solution was cooled to room temperature, and a 25 v/v % aqueous ammonia solution was added to adjust the pH to 9.0. Next, the pH-adjusted solution was filtered through a 100-mesh wire mesh, and an appropriate amount of ion-exchange water was added to adjust the solid content to 40.0% by mass, thereby obtaining resin C-1 having a carbonyl group.

<Production of semicarbazide composition>

[Example 1-1]

(Production of semicarbazide composition S-a1)

40.0 g of the semicarbazide composition S-c1 synthesized in Synthesis Example 2-1 and 40 g of tetrahydrofuran (hereinafter abbreviated as "THF") were weighed into a flask equipped with a reflux condenser, a dropping tank, a thermometer and a stirrer, and stirred under a nitrogen atmosphere. Then, while maintaining the inside of the flask at 20°C, a mixture of 20.0 g of the isocyanate compound N-1 synthesized in Synthesis Example 1-1 and 20 g of THF was added to the flask over 1 hour. After the addition, the mixture was stirred for 20 minutes, 0.8 g of levulinic acid was added, and the mixture was stirred for 10 minutes. Then, 0.8 g of dimethylethanolamine was added and stirred for 10 minutes to obtain a reaction solution containing a semicarbazide compound.

The entire amount of the reaction solution thus obtained was transferred to an eggplant flask, and after adding

140 g of water, the pressure was reduced under vacuum at room temperature to remove THF. Appropriate amounts of water and butyl cellosolve were added to the remaining solution to adjust the solid content to 30% by mass and the butyl cellosolve concentration to 15% by mass to obtain semicarbazide composition S-a1.

[Example 1-2]

(Production of semicarbazide composition S-a2)

40.0 g of the semicarbazide composition S-c1 synthesized in Synthesis Example 2-1 and 40 g of THF were weighed into a flask equipped with a reflux condenser, a dropping tank, a thermometer and a stirrer, and stirred under a nitrogen atmosphere. Then, while maintaining the inside of the flask at 20°C, a mixture of 16.3 g of the isocyanate compound N-2 synthesized in Synthesis Example 1-2 and 16.3 g of THF was added to the flask over 1 hour. After the addition, the mixture was stirred for 20 minutes, 1.2 g of levulinic acid was added, and the mixture was stirred for 10 minutes. Then, 1.2 g of dimethylethanolamine was added and stirred for 10 minutes to obtain a reaction solution containing a semicarbazide compound.

The entire amount of the reaction solution thus obtained was transferred to an eggplant flask, and after adding 140 g of water, the pressure was reduced under vacuum at room temperature to remove THF. Appropriate amounts of water and butyl cellosolve were added to the remaining solution to adjust the solid content to 30% by mass and the butyl cellosolve concentration to 15% by mass to obtain semicarbazide composition S-a2.

[Example 1-3]

(Production of semicarbazide composition S-a3)

40.0 g of the semicarbazide composition S-c1 synthesized in Synthesis Example 2-1 and 40 g of THF were weighed into a flask equipped with a reflux condenser, a dropping tank, a thermometer and a stirrer, and stirred under a nitrogen atmosphere. Then, while maintaining the inside of the flask at 20°C, a mixture of 25.0 g of the isocyanate compound N-3 synthesized in Synthesis Example 1-3 and 25.0 g of THF was added to the flask over 1 hour. After the addition, the mixture was stirred for 20 minutes, 0.4 g of levulinic acid was added, and the mixture was stirred for 10 minutes. Then, 0.4 g of dimethylethanolamine was added and stirred for 10 minutes to obtain a reaction solution containing a semicarbazide compound.

The entire amount of the reaction solution thus obtained was transferred to an eggplant flask, and after adding 140 g of water, the pressure was reduced under vacuum at room temperature to remove THF. Appropriate amounts of water and butyl cellosolve were added to the remaining solution to adjust the solid content to 30% by mass and the butyl cellosolve concentration to 15% by mass to obtain semicarbazide composition S-a3.

[Example 1-4]

(Production of semicarbazide composition S-a4)

40.0 g of the semicarbazide composition S-c1 synthesized in Synthesis Example 2-1 and 40 g of THF were weighed into a flask equipped with a reflux condenser, a dropping tank, a thermometer and a stirrer, and stirred under a nitrogen atmosphere. Then, while maintaining the inside of the flask at 20°C, a mixture of 15.5 g of the isocyanate compound N-1 synthesized in Synthesis Example 1-1 and 15.5 g of THF was added to the flask over 1 hour. After the addition, the mixture was stirred for 20 minutes, 1.3 g of levulinic acid was added, and the mixture was stirred for 10 minutes. Then, 1.3 g of dimethylethanolamine was added and stirred for 10 minutes to obtain a reaction solution containing a semicarbazide compound.

The entire amount of the reaction solution thus obtained was transferred to an eggplant flask, and after adding 140 g of water, the pressure was reduced under vacuum at room temperature to remove THF. Appropriate amounts of water and butyl cellosolve were added to the remaining solution to adjust the solid content to 30% by mass and the butyl cellosolve concentration to 15% by mass to obtain semicarbazide composition S-a4.

[Example 1-5]

(Production of semicarbazide composition S-a5)

40.0 g of the semicarbazide composition S-c1 synthesized in Synthesis Example 2-1 and 40 g of THF were weighed into a flask equipped with a reflux condenser, a dropping tank, a thermometer and a stirrer, and stirred under

a nitrogen atmosphere. Then, while maintaining the inside of the flask at 20°C, a mixture of 16.0 g of the isocyanate compound N-4 synthesized in Synthesis Example 1-4 and 16.0 g of THF was added to the flask over 1 hour. After the addition, the mixture was stirred for 20 minutes, 1.3 g of levulinic acid was added, and the mixture was stirred for 10 minutes. Then, 1.3 g of dimethylethanolamine was added and stirred for 10 minutes to obtain a reaction solution containing a semicarbazide compound.

The entire amount of the reaction solution thus obtained was transferred to an eggplant flask, and after adding 140 g of water, the pressure was reduced under vacuum at room temperature to remove THF. Appropriate amounts of water and butyl cellosolve were added to the remaining solution to adjust the solid content to 30% by mass and the butyl cellosolve concentration to 15% by mass to obtain semicarbazide composition S-a6.

[Example 1-6]

(Production of semicarbazide composition S-a6)

40.0 g of the semicarbazide composition S-c1 synthesized in Synthesis Example 2-1 and 40 g of THF were weighed into a flask equipped with a reflux condenser, a dropping tank, a thermometer and a stirrer, and stirred under a nitrogen atmosphere. Then, while maintaining the inside of the flask at 20°C, a mixture of 22.0 g of the isocyanate compound N-5 synthesized in Synthesis Example 1-5 and 22.0 g of THF was added to the flask over 1 hour. After the addition, the mixture was stirred for 20 minutes, 1.6 g of levulinic acid was added, and the mixture was stirred for 10 minutes. Then, 1.6 g of dimethylethanolamine was added and stirred for 10 minutes to obtain a reaction solution containing a semicarbazide compound.

The entire amount of the reaction solution thus obtained was transferred to an eggplant flask, and after adding 140 g of water, the pressure was reduced under vacuum at room temperature to remove THF. Appropriate amounts of water and butyl cellosolve were added to the remaining solution to adjust the solid content to 30% by mass and the butyl cellosolve concentration to 15% by mass to obtain semicarbazide composition S-a6.

[Example 1-7]

(Production of semicarbazide composition S-a7)

40.0 g of the semicarbazide composition S-c1 synthesized in Synthesis Example 2-1 and 40 g of THF were weighed into a flask equipped with a reflux condenser, a dropping tank, a thermometer and a stirrer, and stirred under a nitrogen atmosphere. Then, while maintaining the inside of the flask at 20°C, a mixture of 17.5 g of the isocyanate compound N-6 synthesized in Synthesis Example 1-6 and 17.5 g of THF was added to the flask over 1 hour. After the addition, the mixture was stirred for 20 minutes, 1.2 g of levulinic acid was added, and the mixture was stirred for 10 minutes. Then, 1.2 g of dimethylethanolamine was added and stirred for 10 minutes to obtain a reaction solution containing a semicarbazide compound.

The entire amount of the reaction solution thus obtained was transferred to an eggplant flask, and after adding 140 g of water, the pressure was reduced under vacuum at room temperature to remove THF. Appropriate amounts of water and butyl cellosolve were added to the remaining solution to adjust the solid content to 30% by mass and the butyl cellosolve concentration to 15% by mass to obtain semicarbazide composition S-a7.

[Example 1-8]

(Production of semicarbazide composition S-a8)

40.0 g of the semicarbazide composition S-c1 synthesized in Synthesis Example 2-1 and 40 g of THF were weighed into a flask equipped with a reflux condenser, a dropping tank, a thermometer and a stirrer, and stirred under a nitrogen atmosphere. Then, while maintaining the inside of the flask at 20°C, a mixture of 35.0 g of the isocyanate compound N-7 synthesized in Synthesis Example 1-7 and 35.0 g of THF was added to the flask over 1 hour. After the addition, the mixture was stirred for 20 minutes, 1.3 g of levulinic acid was added, and the mixture was stirred for 10 minutes. Then, 1.3 g of dimethylethanolamine was added and stirred for 10 minutes to obtain a reaction solution containing a semicarbazide compound.

The entire amount of the reaction solution thus obtained was transferred to an eggplant flask, and after adding 140 g of water, the pressure was reduced under vacuum at room temperature to remove THF. Appropriate amounts of water and butyl cellosolve were added to the remaining solution to adjust the solid content to 30% by mass and the butyl cellosolve concentration to 15% by mass to obtain semicarbazide composition S-a8.

[Example 1-9]

(Production of semicarbazide composition S-a9)

40.0 g of the semicarbazide composition S-c1 synthesized in Synthesis Example 2-1 and 40 g of THF were weighed into a flask equipped with a reflux condenser, a dropping tank, a thermometer and a stirrer, and stirred under a nitrogen atmosphere. Then, while maintaining the inside of the flask at 20°C, a mixture of 19.0 g of the isocyanate compound N-6 synthesized in Synthesis Example 1-6 and 19.0 g of THF was added to the flask over 1 hour. After the addition, the mixture was stirred for 20 minutes, 1.2 g of levulinic acid was added, and the mixture was stirred for 10 minutes. Then, 1.2 g of dimethylethanolamine was added and stirred for 10 minutes to obtain a reaction solution containing a semicarbazide compound.
The entire amount of the reaction solution thus obtained was transferred to an eggplant flask, and after adding 140 g of water, the pressure was reduced under vacuum at room temperature to remove THF. Appropriate amounts of water and butyl cellosolve were added to the remaining solution to adjust the solid content to 30% by mass and the butyl cellosolve concentration to 15% by mass to obtain semicarbazide composition S-a9.

[Example 1-10]

(Production of semicarbazide composition S-a10)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 54.1 g of the isocyanate compound N-1 synthesized in Synthesis Example 1-1 and 54.1 g of THF was added to the flask over 1 hour. After the addition, the mixture was stirred for 20 minutes, a mixture of 8.5 g of IPDI and 8.5 g of cyclohexane was added, and the mixture was stirred for 10 minutes to obtain a reaction solution containing a semicarbazide compound.
The entire amount of the reaction solution thus obtained was transferred to an eggplant flask, and after adding 280 g of water, the pressure was reduced under vacuum at room temperature to remove THF and cyclohexane. An appropriate amount of water was added to the remaining solution to adjust the solid content to 30% by mass, thereby obtaining semicarbazide composition S-a10.

[Example 1-11]

(Production of semicarbazide composition S-a11)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 43.3 g of the isocyanate compound N-2 synthesized in Synthesis Example 1-2 and 43.3 g of THF was added to the flask over 1 hour. After the addition, the mixture was stirred for 20 minutes, a mixture of 8.5 g of IPDI and 8.5 g of cyclohexane was added, and the mixture was stirred for 10 minutes to obtain a reaction solution containing a semicarbazide compound.
The entire amount of the reaction solution thus obtained was transferred to an eggplant flask, and after adding 280 g of water, the pressure was reduced under vacuum at room temperature to remove THF and cyclohexane. An appropriate amount of water was added to the remaining solution to adjust the solid content to 30% by mass, thereby obtaining semicarbazide composition S-a11.

[Example 1-12]

(Production of semicarbazide composition S-a12)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 64.5 g of the isocyanate compound N-3 synthesized in Synthesis Example 1-3 and 64.5 g of THF was added to the flask over 1 hour. After the addition, the mixture was stirred for 20 minutes, a mixture of 8.5 g of IPDI and 8.5 g of cyclohexane was added, and the mixture was stirred for 10 minutes to obtain a reaction solution containing a semicarbazide compound.
The entire amount of the reaction solution thus obtained was transferred to an eggplant flask, and after adding 280 g of water, the pressure was reduced under vacuum at room temperature to remove THF and cyclohexane. An

appropriate amount of water was added to the remaining solution to adjust the solid content to 30% by mass, thereby obtaining semicarbazide composition S-a12.

[Example 1-13]

(Production of semicarbazide composition S-a13)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 52.2 g of the isocyanate compound N-4 synthesized in Synthesis Example 1-4 and 52.2 g of THF was added to the flask over 1 hour. After the addition, the mixture was stirred for 20 minutes, a mixture of 8.5 g of IPDI and 8.5 g of cyclohexane was added, and the mixture was stirred for 10 minutes to obtain a reaction solution containing a semicarbazide compound.

The entire amount of the reaction solution thus obtained was transferred to an eggplant flask, and after adding 280 g of water, the pressure was reduced under vacuum at room temperature to remove THF and cyclohexane. Appropriate amounts of water and butyl cellosolve were added to the remaining solution to adjust the solid content to 30% by mass and the butyl cellosolve concentration to 10% by mass to obtain semicarbazide composition S-a13.

[Example 1-14]

(Production of semicarbazide composition S-a14)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 87.1 g of the isocyanate compound N-5 synthesized in Synthesis Example 1-5 and 87.1 g of THF was added to the flask over 1 hour. After the addition, the mixture was stirred for 20 minutes, a mixture of 8.5 g of IPDI and 8.5 g of cyclohexane was added, and the mixture was stirred for 10 minutes to obtain a reaction solution containing a semicarbazide compound.

The entire amount of the reaction solution thus obtained was transferred to an eggplant flask, and after adding 280 g of water, the pressure was reduced under vacuum at room temperature to remove THF and cyclohexane. Appropriate amounts of water and butyl cellosolve were added to the remaining solution to adjust the solid content to 30% by mass and the butyl cellosolve concentration to 10% by mass to obtain semicarbazide composition S-a14.

[Example 1-15]

(Production of semicarbazide composition S-a15)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 47.3 g of the isocyanate compound N-6 synthesized in Synthesis Example 1-6 and 47.3 g of THF was added to the flask over 1 hour. After the addition, the mixture was stirred for 20 minutes, a mixture of 8.5 g of IPDI and 8.5 g of cyclohexane was added, and the mixture was stirred for 10 minutes to obtain a reaction solution containing a semicarbazide compound.

The entire amount of the reaction solution thus obtained was transferred to an eggplant flask, and after adding 280 g of water, the pressure was reduced under vacuum at room temperature to remove THF and cyclohexane. An appropriate amount of water was added to the remaining solution to adjust the solid content to 30% by mass, thereby obtaining semicarbazide composition S-a15.

[Example 1-16]

(Production of semicarbazide composition S-a16)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 93.3 g of the isocyanate compound N-7 synthesized in Synthesis Example 1-7 and 93.3 g of THF was added to the flask over 1 hour. After the addition, the mixture was stirred for 20 minutes, a mixture of 8.5 g of IPDI and 8.5 g of cyclohexane was added, and the mixture was stirred for 10 minutes to obtain a reaction solution containing a semicarbazide compound.

The entire amount of the reaction solution thus obtained was transferred to an eggplant flask, and after adding 280 g of water, the pressure was reduced under vacuum at room temperature to remove THF and cyclohexane. Appropriate amounts of water and butyl cellosolve were added to the remaining solution to adjust the solid content to 30% by mass and the butyl cellosolve concentration to 10% by mass to obtain semicarbazide composition S-a16.

[Example 1-17]

(Production of semicarbazide composition S-a17)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 54.1 g of the isocyanate compound N-1 synthesized in Synthesis Example 1-1 and 54.1 g of THF was added to the flask over 1 hour. After the addition, the mixture was stirred for 20 minutes, a mixture of 8.5 g of IPDI and 8.5 g of cyclohexane was added, and the mixture was stirred for 10 minutes. Next, 4.4 g of levulinic acid was added with stirring, and after 10 minutes, 4.4 g of dimethylethanolamine was added and stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass, thereby obtaining a semicarbazide composition S-a17.

[Example 1-18]

(Production of semicarbazide composition S-a18)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 54.1 g of the isocyanate compound N-1 synthesized in Synthesis Example 1-1 and 54.1 g of THF was added to the flask over 1 hour. After the addition, it was stirred for 20 minutes, and a mixture of 4.4 g of IPDI and 4.4 g of cyclohexane was added and stirred for 10 minutes. Next, a mixture of 0.5 g of HD1 and 0.5 g of cyclohexane was added with stirring and then stirred for 10 minutes to obtain a reaction solution containing a semicarbazide compound.

The entire amount of the reaction solution thus obtained was transferred to an eggplant flask, and after adding 280 g of water, the pressure was reduced under vacuum at room temperature to remove THF and cyclohexane. Appropriate amounts of water and butyl cellosolve were added to the remaining solution to adjust the solid content to 30% by mass and the butyl cellosolve concentration to 5% by mass to obtain semicarbazide composition S-a18.

[Example 1-19]

(Production of semicarbazide composition S-a19)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 26.0 g of the isocyanate compound N-2 synthesized in Synthesis Example 1-2 and 26.0 g of THF was added to the flask over 1 hour. After the addition, the mixture was stirred for 20 minutes, and a mixture of 15.0 g of IPDI and 15.0 g of cyclohexane was added and stirred for 10 minutes. Next, 0.3 g of levulinic acid was added with stirring, and after 10 minutes, 0.3 g of dimethylethanolamine was added and stirred for 10 minutes to obtain a reaction solution containing a semicarbazide compound.

The entire amount of the reaction solution thus obtained was transferred to an eggplant flask, and after adding 280 g of water, the pressure was reduced under vacuum at room temperature to remove THF and cyclohexane. Appropriate amounts of water and butyl cellosolve were added to the remaining solution to adjust the solid content to 30% by mass and the butyl cellosolve concentration to 10% by mass to obtain semicarbazide composition S-a19.

[Example 1-20]

(Production of semicarbazide composition S-a20)

55.0 g of water, 25.0 g of THF, and 9.4 g of hydrazine monohydrate were weighed into a flask equipped with

a reflux condenser, a dropping tank, a thermometer and a stirrer, and stirred under a nitrogen atmosphere. Then, while maintaining the inside of the flask at 20°C, a mixture of 56.0 g of the isocyanate compound N-7 synthesized in Synthesis Example 1-7 and 56.0 g of THF was added to the flask over 1 hour. After the addition, the mixture was stirred for 20 minutes, and a mixture of 19.0 g of IPDI and 19.0 g of cyclohexane was added and stirred for 10 minutes. Next, 0.5 g of levulinic acid was added with stirring, and after 10 minutes, 0.5 g of dimethylethanolamine was added and stirred for 10 minutes to obtain a reaction solution containing a semicarbazide compound.

The entire amount of the reaction solution thus obtained was transferred to an eggplant flask, and after adding 280 g of water, the pressure was reduced under vacuum at room temperature to remove THF and cyclohexane. Appropriate amounts of water and butyl cellosolve were added to the remaining solution to adjust the solid content to 30% by mass and the butyl cellosolve concentration to 10% by mass to obtain semicarbazide composition S-a20.

[Example 1-21]

(Production of semicarbazide composition S-a21)

55.0 g of water, 25.0 g of THF, and 9.4 g of hydrazine monohydrate were weighed into a flask equipped with a reflux condenser, a dropping tank, a thermometer and a stirrer device, and stirred under a nitrogen atmosphere. Then, while maintaining the inside of the flask at 20°C, 42.8 g of the isocyanate compound N-1 synthesized in Synthesis Example 1-1, 10.0 g of the isocyanate compound N-5 synthesized in Synthesis Example 1-5, and 52.8 g of THF was added to the flask over 1 hour. After the addition, the mixture was stirred for 20 minutes, a mixture of 8.5 g of IPDI and 8.5 g of cyclohexane was added, and the mixture was stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. Appropriate amounts of water and butyl cellosolve were added to the remaining solution to adjust the solid content to 30% by mass and the butyl cellosolve concentration to be 5% by mass to obtain semicarbazide composition S-a21.

[Example 1-22]

(Production of semicarbazide composition S-a22)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 39.0 g of the isocyanate compound N-7 synthesized in Synthesis Example 1-7 and 39.0 g of THF was added to the flask over 1 hour. After the addition, the mixture was stirred for 20 minutes, and a mixture of 20.0 g of IPDI and 20.0 g of cyclohexane was added and stirred for 10 minutes. Next, 0.1 g of levulinic acid was added with stirring, and after 10 minutes, 0.1 g of dimethylethanolamine was added and stirred for 10 minutes to obtain a reaction solution containing a semicarbazide compound.

The entire amount of the reaction solution thus obtained was transferred to an eggplant flask, and after adding 280 g of water, the pressure was reduced under vacuum at room temperature to remove THF and cyclohexane. Appropriate amounts of water and butyl cellosolve were added to the remaining solution to adjust the solid content to 30% by mass and the butyl cellosolve concentration to 10% by mass to obtain semicarbazide composition S-a22.

[Example 1-23]

(Production of semicarbazide composition S-a23)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 49.2 g of the isocyanate compound N-2 synthesized in Synthesis Example 1-2 and 49.2 g of THF was added to the flask over 1 hour. After the addition, it was stirred for 20 minutes, and a mixture of 1.7 g of IPDI and 1.7 g of cyclohexane was added and stirred for 10 minutes. Then, a mixture of 0.3 g of HDI and 0.3 g of cyclohexane was added while stirring, and the reaction solution containing the semicarbazide compound was obtained by stirring for 10 minutes.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass, thereby obtaining a semicarbazide composition Sa23.

[Example 1-24]

(Production of semicarbazide composition S-a24)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 70.0 g of the isocyanate compound N-3 synthesized in Synthesis Example 1-3 and 70.0 g of THF was added to the flask over 1 hour. After the addition, the mixture was stirred for 20 minutes, and a mixture of 7.0 g of IPDI and 7.0 g of cyclohexane was added and stirred for 10 minutes. Subsequently, 3.0 g of levulinic acid was added with stirring, and after 10 minutes, 3.0 g of dimethylethanolamine was added and stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass to obtain a semicarbazide composition S-a24.

[Example 1-25]

(Production of semicarbazide composition S-a25)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, a mixture of 15.0 g of the isocyanate compound N-3 synthesized in Synthesis Example 1-3 and 15.0 g of THF was added to the flask over 1 hour while maintaining the inside of the flask at 20°C. After the addition, the mixture was stirred for 20 minutes, a mixture of 20.0 g of IPDI and 20.0 g of cyclohexane was added, and the mixture was stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. Appropriate amounts of water and butyl cellosolve were added to the remaining solution to adjust the solid content to 30% by mass and the butyl cellosolve concentration to 10% by mass to obtain semicarbazide composition S-a25.

[Example 1-26]

(Production of semicarbazide composition S-a26)

55.0 g of water, 25.0 g of THF, and 9.4 g of hydrazine monohydrate were weighed into a flask equipped with a reflux condenser, a dropping tank, a thermometer and a stirrer, and stirred under a nitrogen atmosphere. Then, while maintaining the inside of the flask at 20°C, a mixture of 7.5 g of the isocyanate compound N-3 synthesized in Synthesis Example 1-3 and 7.5 g of THF was added to the flask over 1 hour. After the addition, the mixture was stirred for 20 minutes, a mixture of 23.3 g of IPDI and 23.3 g of cyclohexane was added, and the mixture was stirred for 10 minutes to obtain a reaction solution containing a semicarbazide compound.

The entire amount of the reaction solution thus obtained was transferred to an eggplant flask, and after adding 280 g of water, the pressure was reduced under vacuum at room temperature to remove THF and cyclohexane. Appropriate amounts of water and butyl cellosolve were added to the remaining solution to adjust the solid content to 30% by mass and the butyl cellosolve concentration to 15% by mass to obtain semicarbazide composition S-a26.

[Example 1-27]

(Production of semicarbazide composition S-a27)

55.0 g of water, 25.0 g of THF, and 9.4 g of hydrazine monohydrate were weighed into a flask equipped with a reflux condenser, a dropping tank, a thermometer, and a stirring device, and stirred under a nitrogen atmosphere. Next, a mixture of 59.0 g of the isocyanate compound N-1 synthesized in Synthesis Example 1-1 and 59.0 g of THF was added to the flask over 1 hour while maintaining the inside of the flask at 20°C. After the addition, the mixture was stirred for 20 minutes, a mixture of 1.9 g of HDI and 1.9 g of cyclohexane was added, and the mixture was stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane.

An appropriate amount of water was added to the remaining solution to adjust the solid content to 30% by mass, thereby obtaining a semicarbazide composition S-a27.

[Example 1-28]

(Production of semicarbazide composition S-a28)

55.0 g of water, 25.0 g of THF, and 9.4 g of hydrazine monohydrate were weighed into a flask equipped with a reflux condenser, a dropping tank, a thermometer, and a stirrer, and stirred under a nitrogen atmosphere. Then, while maintaining the inside of the flask at 20°C, a mixture of 4.0 g of the isocyanate compound N-3 synthesized in Synthesis Example 1-3 and 4.0 g of THF was added to the flask over 1 hour. After the addition, the mixture was stirred for 20 minutes, a mixture of 22.0 g of IPDI and 22.0 g of cyclohexane was added, and the mixture was stirred for 10 minutes to obtain a reaction solution containing a semicarbazide compound.

The entire amount of the reaction solution obtained was transferred to an eggplant flask, 140 g of water was added, and then the pressure was reduced under vacuum at room temperature to remove THF and cyclohexane. Appropriate amounts of water and butyl cellosolve were added to the remaining solution to adjust the solid content to 30% by mass and the butyl cellosolve concentration to be 15% by mass to obtain semicarbazide composition S-a28.

[Example 1-29]

(Production of semicarbazide composition S-a29)

55.0 g of water, 25.0 g of THF, and 9.4 g of hydrazine monohydrate were weighed into a flask equipped with a reflux condenser, a dropping tank, a thermometer, and a stirrer, and stirred under a nitrogen atmosphere. Then, while maintaining the inside of the flask at 20°C, a mixture of 12.0 g of the isocyanate compound N-7 synthesized in Synthesis Example 1-7 and 12.0 g of THF was added to the flask over 1 hour. After the addition, it was stirred for 20 minutes, and a mixture of 18.8 g of IPDI and 18.8 g of cyclohexane was added and stirred for 10 minutes. Next, 0.2 g of levulinic acid was added with stirring, and after 10 minutes, 0.2 g of dimethylethanolamine was added and stirred for 10 minutes to obtain a reaction solution containing a semicarbazide compound.

The entire amount of the reaction solution thus obtained was transferred to an eggplant flask, 140 g of water was added, and then the pressure was reduced under vacuum at room temperature to remove THF and cyclohexane. Appropriate amounts of water and butyl cellosolve were added to the remaining solution to adjust the solid content to 30% by mass and the butyl cellosolve concentration to 15% by mass to obtain semicarbazide composition S-a29.

[Example 1-30]

(Production of semicarbazide composition S-a30)

55.0 g of water, 25.0 g of THF, and 9.4 g of hydrazine monohydrate were weighed into a flask equipped with a reflux condenser, a dropping tank, a thermometer, and a stirrer, and stirred under a nitrogen atmosphere. Then, while maintaining the inside of the flask at 20°C, a mixture of 73.0 g of the isocyanate compound N-3 synthesized in Synthesis Example 1-3 and 73.0 g of THF was added to the flask over 1 hour. After the addition, it was stirred for 20 minutes, and a mixture of 5.9 g of IPDI and 5.9 g of cyclohexane was added and stirred for 10 minutes. Next, 4.2 g of levulinic acid was added with stirring, and after 10 minutes, 4.2 g of dimethylethanolamine was added and stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass, thereby obtaining semicarbazide composition Sa30.

[Comparative Example 1-1]

(Production of semicarbazide composition S-b1)

55.0 g of water, 25.0 g of THF, and 62.0 g of hydrazine monohydrate were weighed into a flask equipped with a reflux condenser, a dropping tank, a thermometer, and a stirrer, and stirred under a nitrogen atmosphere. Next, a mixture of 42.8 g of the isocyanate compound N-1 synthesized in Synthesis Example 1-1 and 42.8 g of THF was added to the flask over 1 hour while maintaining the inside of the flask at 20°C. After the addition, the mixture was stirred for

30 minutes to obtain a reaction solution containing the semicarbazide compound.

The entire amount of the reaction solution thus obtained was transferred to an eggplant flask, and the pressure was reduced under heating vacuum to remove THF and residual hydrazine. An appropriate amount of water was added to the remaining nonvolatile matter to adjust the solid content to 30% by mass, thereby obtaining a semicarbazide composition S-b1.

[Comparative Example 1-2]

(Production of semicarbazide composition S-b2)

55.0 g of water, 25.0 g of THF, and 9.4 g of hydrazine monohydrate were weighed into a flask equipped with a reflux condenser, a dropping tank, a thermometer, and a stirrer, and stirred under a nitrogen atmosphere. Then, while maintaining the inside of the flask at 20°C, a mixture of 4.0 g of the isocyanate compound N-3 synthesized in Synthesis Example 1-3 and 4.0 g of THF was added to the flask over 1 hour. After the addition, the mixture was stirred for 20 minutes, a mixture of 22.3 g of IPDI and 22.3 g of cyclohexane was added, and the mixture was stirred for 10 minutes to obtain a reaction solution containing a semicarbazide compound.

The entire amount of the reaction solution thus obtained was transferred to an eggplant flask, 140 g of water was added, and then the pressure was reduced under vacuum at room temperature to remove THF and cyclohexane. Appropriate amounts of water and butylcellosolve were added to the remaining solution to adjust the solid content to 30% by mass and the butylcellosolve concentration to 15% by mass to obtain a semicarbazide composition S-b2.

[Example 1-31]

(Production of semicarbazide composition S-a31)

40.0 g of water, 40.0 g of THF, and 9.4 g of hydrazine monohydrate were weighed into a flask equipped with a reflux condenser, a dropping tank, a thermometer, and a stirrer, and stirred under a nitrogen atmosphere. Then, while maintaining the inside of the flask at 20°C, a mixture of 24.1 g of the isocyanate compound N-2 synthesized in Synthesis Example 1-2 and 24.1 g of THF was added to the flask over 1 hour. After the addition, the mixture was stirred for 20 minutes, and a mixture of 18.2 g of IPDI and 18.2 g of cyclohexane was added and stirred for 10 minutes. Then, 1.2 g of levulinic acid was added with stirring, and after 10 minutes, 1.2 g of dimethylethanolamine was added and stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass, thereby obtaining a semicarbazide composition S-a31.

[Example 1-32]

(Production of semicarbazide composition S-a32)

40.0 g of water, 40.0 g of THF, and 9.4 g of hydrazine monohydrate were weighed into a flask equipped with a reflux condenser, a dropping tank, a thermometer, and a stirrer, and stirred under a nitrogen atmosphere. Then, while maintaining the inside of the flask at 20°C, a mixture of 23.0 g of the isocyanate compound N-2 synthesized in Synthesis Example 1-2 and 23.0 g of THF was added to the flask over 1 hour. After the addition, the mixture was stirred for 20 minutes, and a mixture of 20.1 g of IPDI and 20.1 g of cyclohexane was added and stirred for 10 minutes. Then, 1.7 g of levulinic acid was added with stirring, and after 10 minutes, 1.7 g of dimethylethanolamine was added and stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass, thereby obtaining a semicarbazide composition S-a32.

[Example 1-33]

(Production of semicarbazide composition S-a33)

40.0 g of water, 40.0 g of THF, and 9.4 g of hydrazine monohydrate were weighed into a flask equipped with

a reflux condenser, a dropping tank, a thermometer, and a stirrer, and stirred under a nitrogen atmosphere. Then, a mixture of 50.0 g of the isocyanate compound N-1 synthesized in Synthesis Example 1-1 and 50.0 g of THF was added to the flask over 1 hour while maintaining the inside of the flask at 20°C. After the addition, the mixture was stirred for 20 minutes, and a mixture of 11.0 g of IPDI and 11.0 g of cyclohexane was added and stirred for 10 minutes. Then, 2.5 g of levulinic acid was added with stirring, and after 10 minutes, 2.5 g of dimethylethanolamine was added and stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass, thereby obtaining a semicarbazide composition S-a33.

[Example 1-34]

(Production of semicarbazide composition S-a34)

40.0 g of water, 40.0 g of THF, and 9.4 g of hydrazine monohydrate were weighed into a flask equipped with a reflux condenser, a dropping tank, a thermometer, and a stirrer, and stirred under a nitrogen atmosphere. Next, a mixture of 46.0 g of the isocyanate compound N-2 synthesized in Synthesis Example 1-2 and 46.0 g of THF was added to the flask over 1 hour while maintaining the inside of the flask at 20°C. After the addition, the mixture was stirred for 20 minutes, and a mixture of 13.0 g of IPDI and 13.0 g of cyclohexane was added and stirred for 10 minutes. Then, 0.1 g of levulinic acid was added with stirring, and after 10 minutes, 0.1 g of dimethylethanolamine was added and stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass to obtain a semicarbazide composition S-a34.

[Example 1-35]

(Production of semicarbazide composition S-a35)

40.0 g of water, 40.0 g of THF, and 9.4 g of hydrazine monohydrate were weighed into a flask equipped with a reflux condenser, a dropping tank, a thermometer, and a stirrer, and stirred under a nitrogen atmosphere. Next, a mixture of 59.0 g of the isocyanate compound N-4 synthesized in Synthesis Example 1-4 and 59.0 g of THF was added to the flask over 1 hour while maintaining the inside of the flask at 20°C. After the addition, the mixture was stirred for 20 minutes, and a mixture of 7.0 g of IPDI and 7.0 g of cyclohexane was added and stirred for 10 minutes. Then, 0.1 g of levulinic acid was added with stirring, and after 10 minutes, 0.1 g of dimethylethanolamine was added and stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass, thereby obtaining a semicarbazide composition Sa35.

[Example 1-36]

(Production of semicarbazide composition S-a36)

40.0 g of water, 40.0 g of THF, and 9.4 g of hydrazine monohydrate were weighed into a flask equipped with a reflux condenser, a dropping tank, a thermometer, and a stirrer, and stirred under a nitrogen atmosphere. Next, a mixture of 10.0 g of the isocyanate compound N-2 synthesized in Synthesis Example 1-2 and 10.0 g of THF was added to the flask over 1 hour while maintaining the inside of the flask at 20°C. After the addition, the mixture was stirred for 20 minutes, and a mixture of 17.1 g of IPDI and 17.1 g of cyclohexane was added and stirred for 10 minutes. Subsequently, 3.0 g of levulinic acid was added with stirring, and after 10 minutes, 3.0 g of dimethylethanolamine was added and stirred for 10 minutes to obtain a reaction solution containing a semicarbazide compound.

The entire amount of the reaction solution thus obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass, thereby obtaining a semicarbazide composition S-a36.

[Comparative Example 1-3]

(Production of semicarbazide composition S-b3)

40.0 g of water, 40.0 g of THF, and 9.4 g of hydrazine monohydrate were weighed into a flask equipped with a reflux condenser, a dropping tank, a thermometer, and a stirrer, and stirred under a nitrogen atmosphere. Then, while maintaining the inside of the flask at 20°C, a mixture of 44.5 g of the isocyanate compound N-7 synthesized in Synthesis Example 1-7 and 44.5 g of THF was added to the flask over 1 hour. After the addition, it was stirred for 20 minutes, and a mixture of 22.9 g of IPDI and 22.9 g of cyclohexane was added and stirred for 10 minutes. Then, 0.1 g of levulinic acid was added with stirring, and after 10 minutes, 0.1 g of dimethylethanolamine was added and stirred for 10 minutes to obtain a reaction solution containing a semicarbazide compound.

The entire amount of the reaction solution obtained was transferred to an eggplant flask, 280 g of water was added, and then the pressure was reduced under vacuum at room temperature to remove THF and cyclohexane. An appropriate amount of water was added to the remaining solution to adjust the solid content to 30% by mass, thereby obtaining a semicarbazide composition S-b3.

Tables 1 to 6 show the physical properties of each semicarbazide composition obtained in the Examples and the Comparative Examples.

[Table 1]

| | | Ex. 1-1 | Ex. 1-2 | Ex. 1-3 | Ex. 1-4 | Ex. 1-5 | Ex. 1-6 | Ex. 1-7 | Ex. 1-8 | Ex. 1-9 |
|---|---|---|---|---|---|---|---|---|---|---|
| Semicarbazide composition | | S-a1 | S-a2 | S-a3 | S-a4 | S-a5 | S-a6 | S-a7 | S-a8 | S-a9 |
| Composition (g) | N-1 | 20.0 | | | 15.5 | | | | | |
| | N-2 | | 16.3 | | | | | | | |
| | N-3 | | | 25.0 | | | | | | |
| | N-4 | | | | | 16.0 | | | | |
| | N-5 | | | | | | 22.0 | | | |
| | N-6 | | | | | | | 17.5 | | 19.0 |
| | N-7 | | | | | | | | 35.0 | |
| | S-c1 | 40.0 | 40.0 | 40.0 | 40.0 | 40.0 | 40.0 | 40.0 | 40.0 | 40.0 |
| | IPDI | | | | | | | | | |
| | HDI | | | | | | | | | |
| | Levulinic acid | 0.8 | 1.2 | 0.4 | 1.3 | 1.3 | 1.6 | 1.2 | 1.3 | 1.2 |
| | Hydrazine | | | | | | | | | |
| Hydrophilic functional group (a) content (mass%) | | 20.0 | 15.4 | 24.1 | 19.5 | 18.5 | 25.3 | 14.6 | 10.3 | 14.1 |
| Aliphatic isocyanate content (mass%) | | 46.5 | 44.7 | 49.0 | 40.5 | 41.3 | 46.2 | 47.2 | 67.1 | 49.2 |
| Molar ratio (b)/(a) | | 3.6 | 3.7 | 3.4 | 0.6 | 3.1 | 1.7 | 3.8 | 3.7 | 4.9 |

[Table 2]

| | | Ex. 1-10 | Ex. 1-11 | Ex. 1-12 | Ex. 1-13 | Ex. 1-14 | Ex. 1-15 | Ex. 1-16 |
|---|---|---|---|---|---|---|---|---|
| Semicarbazide composition | | S-a10 | S-a11 | S-a12 | S-a13 | S-a14 | S-a15 | S-a16 |
| Composition (g) | N-1 | 54.1 | | | | | | |
| | N-2 | | 43.3 | | | | | |
| | N-3 | | | 64.5 | | | | |
| | N-4 | | | | 52.2 | | | |
| | N-5 | | | | | 87.1 | | |
| | N-6 | | | | | | 47.3 | |
| | N-7 | | | | | | | 93.3 |
| | IPDI | 8.5 | 8.5 | 8.5 | 8.5 | 8.5 | 8.5 | 8.5 |
| | HDI | | | | | | | |
| | Levulinic acid | | | | | | | |
| | Hydrazine | 9.4 | 9.4 | 9.4 | 9.4 | 9.4 | 9.4 | 9.4 |
| Hydrophilic functional group (a) content (mass%) | | 29.0 | 20.9 | 35.7 | 19.9 | 36.9 | 19.4 | 11.2 |
| Aliphatic isocyanate content (mass%) | | 80.1 | 78.6 | 81.0 | 82.1 | 85.4 | 80.6 | 90.5 |
| Molar ratio (b)/(a) | | 1.6 | 2.6 | 1.1 | 2.4 | 0.9 | 2.6 | 2.6 |

[Table 3]

| | | Ex. 1-17 | Ex. 1-18 | Ex. 1-19 | Ex. 1-20 | Ex. 1-21 | Ex. 1-22 |
|---|---|---|---|---|---|---|---|
| Semicarbazide composition | | S-a17 | S-a18 | S-a19 | S-a20 | S-a21 | S-a22 |
| Composition (g) | N-1 | 54.1 | 54.1 | | | 42.8 | |
| | N-2 | | | 26.0 | | | |
| | N-3 | | | | | | |
| | N-4 | | | | | | |
| | N-5 | | | | | 10.0 | |
| | N-6 | | | | | | |
| | N-7 | | | | 56.0 | | 39.0 |
| | IPDI | 8.5 | 4.4 | 15.0 | 19.0 | 8.5 | 20.0 |
| | HDI | | 0.5 | | | | |
| | Levulinic acid | 4.4 | | 0.3 | 0.5 | | 0.1 |
| | Hydrazine | 9.4 | 9.4 | 9.4 | 9.4 | 9.4 | 9.4 |
| Hydrophilic functional group (a) content (mass%) | | 30.1 | 30.6 | 16.0 | 9.5 | 27.1 | 7.9 |
| Aliphatic isocyanate content (mass%) | | 80.0 | 88.7 | 55.6 | 72.0 | 80.2 | 62.9 |
| Molar ratio (b)/(a) | | 1.2 | 0.5 | 3.3 | 5.3 | 1.7 | 7.4 |

[Table 4]

| | | Ex. 1-23 | Ex. 1-24 | Ex. 1-25 | Ex. 1-26 | Ex. 1-27 | Ex. 1-28 | Ex. 1-29 | Ex. 1-30 |
|---|---|---|---|---|---|---|---|---|---|
| Semicarbazide composition | | S-a23 | S-a24 | S-a25 | S-a26 | S-a27 | S-a28 | S-a29 | S-a30 |
| Composition (g) | N-1 | | | | | 59.0 | | | |
| | N-2 | 49.2 | | | | | | | |
| | N-3 | | 70.0 | 15.0 | 7.5 | | 4.0 | | 73.0 |
| | N-4 | | | | | | | | |
| | N-5 | | | | | | | | |
| | N-6 | | | | | | | | |
| | N-7 | | | | | | | 12.0 | |
| | S-c1 | | | | | | | | |
| | IPDI | 1.7 | 7.0 | 20.0 | 23.3 | | 22.0 | 18.8 | 5.9 |
| | HDI | 0.3 | | | | 1.9 | | | |
| | Levulinic acid | | 3.0 | | | | | 0.2 | 4.2 |
| | Hydrazine | 9.4 | 9.4 | 9.4 | 9.4 | 9.4 | 9.4 | 9.4 | 9.4 |
| Hydrophilic functional group (a) content (mass%) | | 24.0 | 39.1 | 16.0 | 8.9 | 32.4 | 5.4 | 4.7 | 40.5 |
| Aliphatic isocyanate content (mass%) | | 94.3 | 84.9 | 29.7 | 15.3 | 100.0 | 9.3 | 35.7 | 87.4 |
| Molar ratio (b)/(a) | | 0.5 | 1.0 | 3.3 | 9.2 | 0.2 | 9.3 | 1.2 | 0.9 |

[Table 5]

| | | Com. Ex. 1-1 | Com. Ex. 1-2 |
|---|---|---|---|
| Semicarbazide composition | | S-b1 | S-b2 |
| Composition (g) | N-1 | 42.8 | |
| | N-2 | | |
| | N-3 | | 4.0 |
| | N-4 | | |
| | N-5 | | |
| | N-6 | | |
| | N-7 | | |
| | S-c1 | | |
| | IPDI | | 22.3 |
| | HDI | | |
| | Levulinic acid | | |
| | Hydrazine | 62.0 | 9.4 |
| Hydrophilic functional group (a) content (mass%) | | 19.1 | 5.4 |
| Aliphatic isocyanate content (mass%) | | 100.0 | 9.2 |
| Molar ratio (b)/(a) | | 0 | 10.4 |

[Table 6]

| | | Ex. 1-31 | Ex. 1-32 | Ex. 1-33 | Ex. 1-34 | Ex. 1-35 | Ex. 1-36 | Com. Ex. 1-3 |
|---|---|---|---|---|---|---|---|---|
| Semicarbazide composition | | S-a31 | S-a32 | S-a33 | S-a34 | S-a35 | S-a36 | S-b3 |
| Composition (g) | N-1 | | | 50.0 | | | | |
| | N-2 | 24.1 | 23.0 | | 46.0 | | 10.0 | |
| | N-3 | | | | | | | |
| | N-4 | | | | | 59.0 | | |
| | N-5 | | | | | | | |
| | N-6 | | | | | | | |
| | N-7 | | | | | | | 44.5 |
| | IPDI | 18.2 | 20.1 | 11.0 | 13.0 | 7.0 | 17.1 | 22.9 |
| | HDI | | | | | | | |
| | Levulinic acid | 1.2 | 1.7 | 2.5 | 0.1 | 0.1 | 3.0 | 0.1 |
| | Hydrazine | 9.4 | 9.4 | 9.4 | 9.4 | 9.4 | 9.4 | 9.4 |
| Hydrophilic functional group (a) content (mass%) | | 16.0 | 16.0 | 30.1 | 19.9 | 20.9 | 16.0 | 7.9 |
| Aliphatic isocyanate content (mass%) | | 48.8 | 45.2 | 74.2 | 71.8 | 86.3 | 29.7 | 62.9 |
| Molar ratio (b)/(a) | | 3.3 | 3.3 | 1.2 | 4.4 | 2.1 | 0.2 | 10.4 |

<Production of water-based coating composition composition>

[Example 2-1]

(Production of water-based coating composition T-a1)

50 g of the resin C-1 obtained in Synthesis Example 3-1, 15.3 g of the semicarbazide composition S-a1 obtained in Example 1-1, and 1.5 g of butyl cellosolve were added to a plastic cup, and an appropriate amount of water was added so that the solid content of the coating material was 33% by mass, and then the mixture was stirred for 10 minutes using a three-one motor. The above mixture was then filtered through a 200-mesh filter cloth to obtain a water-based coating composition T-a1.

[Examples 2-2 to 2-36 and Comparative Examples 2-1 to 2-4]

(Production of water-based coating compositions T-a2 to T-a36 and T-b1 to T-b4)

Each water-based coating composition was obtained in the same manner as in Example 2-1, except that the compositions shown in Tables 7 to 12 were used.

Each of the obtained water-based coating compositions was evaluated according to the evaluation method described above, and the results are shown in Tables 7 to 12.

[Table 7]

| | | | Ex. 2-1 | Ex. 2-2 | Ex. 2-3 | Ex. 2-4 | Ex. 2-5 | Ex. 2-6 | Ex. 2-7 | Ex. 2-8 | Ex. 2-9 |
|---|---|---|---|---|---|---|---|---|---|---|---|
| Water-based coating composition | | | T-a1 | T-a2 | T-a3 | T-a4 | T-a5 | T-a6 | T-a7 | T-a8 | T-a9 |
| Coating composition (g) | Semicarbazide composition | S-a1 | 15.3 | | | | | | | | |
| | | S-a2 | | 15.0 | | | | | | | |
| | | S-a3 | | | 16.6 | | | | | | |
| | | S-a4 | | | | 12.5 | | | | | |
| | | S-a5 | | | | | 11.9 | | | | |
| | | S-a6 | | | | | | 13.7 | | | |
| | | S-a7 | | | | | | | 15.1 | | |
| | | S-a8 | | | | | | | | 22.6 | |
| | | S-a9 | | | | | | | | | 16.0 |
| | Carbonyl group -containing resin (C) | C-1 | 50.0 | 50.0 | 50.0 | 50.0 | 50.0 | 50.0 | 50.0 | 50.0 | 50.0 |
| | Butyl cellosolve | | 1.5 | 1.5 | 1.5 | 1.5 | 1.5 | 1.5 | 1.5 | 1.5 | 1.5 |
| Evaluation | Storage stability | | ◎ | ◎ | ◎ | ◎ | ○ | ○ | ◎ | ◎ | ◎ |
| | Water resistance | | ○ | ○ | ○ | ○ | ◎ | ○ | ○ | ○ | ○ |
| | Coating film elongation (%) | | 202 | 209 | 211 | 189 | 163 | 167 | 188 | 230 | 180 |

[Table 8]

| | | | Ex. 2-10 | Ex. 2-11 | Ex. 2-12 | Ex. 2-13 | Ex. 2-14 | Ex. 2-15 | Ex. 2-16 |
|---|---|---|---|---|---|---|---|---|---|
| Water-based coating composition | | | T-a 10 | T-a11 | T-a12 | T-a13 | T-a14 | T-a15 | T-a16 |
| Coating composition (g) | Semicarbazide composition | S-a10 | 11.8 | | | | | | |
| | | S-a11 | | 10.0 | | | | | |
| | | S-a12 | | | 13.6 | | | | |
| | | S-a13 | | | | 11.4 | | | |
| | | S-a14 | | | | | 17.5 | | |
| | | S-a15 | | | | | | 10.6 | |
| | | S-a16 | | | | | | | 18.6 |
| | | S-a17 | | | | | | | |
| | | S-a18 | | | | | | | |
| | | S-a19 | | | | | | | |
| | | S-a20 | | | | | | | |
| | | S-a21 | | | | | | | |
| | | S-a22 | | | | | | | |
| | Carbonyl group -containing resin (C) | C-1 | 50.0 | 50.0 | 50.0 | 50.0 | 50.0 | 50.0 | 50.0 |
| | Butyl cellosolve | | 1.5 | 1.5 | 1.5 | 1.5 | 1.5 | 1.5 | 1.5 |
| Evaluation | Storage stability | | ◎ | ◎ | ◎ | ○ | ○ | ◎ | ◎ |
| | Water resistance | | ○ | ○ | ○ | ◎ | ○ | ○ | ○ |
| | Coating film elongation (%) | | 211 | 210 | 230 | 171 | 178 | 194 | 244 |

[Table 9]

| | | | Ex. 2-17 | Ex. 2-18 | Ex. 2-19 | Ex. 2-20 | Ex. 2-21 | Ex. 2-22 |
|---|---|---|---|---|---|---|---|---|
| Water-based coating composition | | | T-a 17 | T-a 18 | T-a19 | T-a20 | T-a21 | T-a22 |
| Coating composition (g) | Semicarbazide composition | S-a10 | | | | | | |
| | | S-a11 | | | | | | |
| | | S-a12 | | | | | | |
| | | S-a13 | | | | | | |
| | | S-a14 | | | | | | |
| | | S-a15 | | | | | | |
| | | S-a16 | | | | | | |
| | | S-a17 | 16.5 | | | | | |
| | | S-a18 | | 9.1 | | | | |
| | | S-a19 | | | 8.1 | | | |
| | | S-a20 | | | | 19.2 | | |
| | | S-a21 | | | | | 11.6 | |
| | | S-a22 | | | | | | 12.9 |
| | Carbonyl group -containing resin (C) | C-1 | 50.0 | 50.0 | 50.0 | 50.0 | 50.0 | 50.0 |
| | Butyl cellosolve | | 1.5 | 1.5 | 1.5 | 1.5 | 1.5 | 1.5 |
| Evaluation | Storage stability | | ◎ | ◎ | ◎ | ○ | ◎ | ○ |
| | Water resistance | | ○ | ○ | ○ | ◎ | ○ | ◎ |
| | Coating film elongation (%) | | 208 | 202 | 200 | 233 | 205 | 212 |

[Table 10]

| | | | Ex. 2-23 | Ex. 2-24 | Ex. 2-25 | Ex. 2-26 | Ex. 2-27 | Ex. 2-28 | Ex. 2-29 | Ex. 2-30 |
|---|---|---|---|---|---|---|---|---|---|---|
| Water-based coating composition | | | T-a23 | T-a24 | T-a25 | T-a26 | T-a27 | T-a28 | T-a29 | T-a30 |
| Coating composition (g) | Semicarbazide composition | S-a23 | 7.8 | | | | | | | |
| | | S-a24 | | 18.1 | | | | | | |
| | | S-a25 | | | 6.4 | | | | | |
| | | S-a26 | | | | 6.2 | | | | |
| | | S-a27 | | | | | 8.9 | | | |
| | | S-a28 | | | | | | 4.9 | | |
| | | S-a29 | | | | | | | 5.0 | |
| | | S-a30 | | | | | | | | 20.1 |
| | | S-b1 | | | | | | | | |
| | | S-b2 | | | | | | | | |
| | | S-c1 | | | | | | | | |
| | Carbonyl group -containing resin (C) | C-1 | 50.0 | 50.0 | 50.0 | 50.0 | 50.0 | 50.0 | 50.0 | 50.0 |
| | Butyl cellosolve | | 1.5 | 1.5 | 1.5 | 1.5 | 1.5 | 1.5 | 1.5 | 1.5 |
| Evaluation | Storage stability | | ◎ | ◎ | ◎ | ◎ | ◎ | ○ | △ | ◎ |
| | Water resistance | | ○ | ○ | ○ | ◎ | △ | ◎ | ◎ | △ |
| | Coating film elongation (%) | | 225 | 235 | 189 | 145 | 244 | 105 | 178 | 241 |

[Table 11]

| | | | Com. Ex. 2-1 | Com. Ex. 2-2 | Com. Ex. 2-3 |
|---|---|---|---|---|---|
| Water-based coating composition | | | T-b1 | T-b2 | T-b3 |
| Coating composition (g) | Semicarbazide composition | S-a23 | | | |
| | | S-a24 | | | |
| | | S-a25 | | | |
| | | S-a26 | | | |
| | | S-a27 | | | |
| | | S-a28 | | | |
| | | S-a29 | | | |
| | | S-a30 | | | |
| | | S-b1 | 9.1 | | |
| | | S-b2 | | 4.9 | |
| | | S-c 1 | | | 2.2 |
| | Carbonyl group -containing resin (C) | C-1 | 50.0 | 50.0 | 50.0 |
| | Butyl cellosolve | | 1.5 | 1.5 | 1.5 |
| Evaluation | Storage stability | | ◎ | × | ◎ |
| | Water resistance | | × | ◎ | ○ |
| | Coating film elongation (%) | | 249 | 110 | 81 |

[Table 12]

| | | | Ex. 2-31 | Ex. 2-32 | Ex. 2-33 | Ex. 2-34 | Ex. 2-35 | Ex. 2-36 | Com. Ex. 2-4 |
|---|---|---|---|---|---|---|---|---|---|
| Water-based coating composition | | | T-a31 | T-a32 | T-a33 | T-a34 | T-a35 | T-a36 | T-b4 |
| Coating composition (g) | Semicarbazide composition | S-a31 | 10.9 | | | | | | |
| | | S-a32 | | 13.3 | | | | | |
| | | S-a33 | | | 15.6 | | | | |
| | | S-a34 | | | | 17.6 | | | |
| | | S-a35 | | | | | 13.0 | | |
| | | S-a36 | | | | | | 5.6 | |
| | | S-b3 | | | | | | | 19.8 |
| | Carbonyl group -containing resin (C) | C-1 | 50.0 | 50.0 | 50.0 | 50.0 | 50.0 | 50.0 | 50.0 |
| | Butyl cellosolve | | 1.5 | 1.5 | 1.5 | 1.5 | 1.5 | 1.5 | 1.5 |
| Evaluation | Storage stability | | ◎ | ◎ | ◎ | ◎ | ◎ | ◎ | × |
| | Water resistance | | ○ | ○ | ○ | ◎ | ◎ | △ | ○ |
| | Coating film elongation (%) | | 198 | 192 | 202 | 201 | 199 | 188 | 210 |

As shown in Tables 7 to 12, in the water-based coating compositions T-a1 to T-a36 (Examples 2-1 to 2-36) using the semicarbazide compositions S-a1 to S-a36 (Examples 1-1 to 1-36) containing a semicarbazide compound (S) having a hydrophilic functional group (a) and a structure (b), wherein the molar ratio (b)/(a) of the structure (b) to the hydrophilic functional group (a) was 0.1 or more and 10.0 or less, storage stability, and water resistance and elongation when formed into a coating film were all good.

Further, in comparison of the water-based coating compositions T-a24 and T-a30 (Examples 2-24 and 2-30) using the semicarbazide compositions S-a24 and S-a30 (Examples 1-24 and 1-30) with different molar ratios (b)/(a) of the structure (b) to the hydrophilic functional group (a), the water-based coating composition Ta-a24 using the semicarbazide composition S-a24 having a molar ratio (b)/(a) of 1.0 or more was superior in water resistance when formed into a coating film.

Further, in comparison of the water-based coating compositions T-a28 and T-a29 (Examples 2-28 and 2-29) using the semicarbazide compositions S-a28 and S-a29 (Examples 1-28 and 1-29) having different contents of the hydrophilic functional group (a), the water-based coating composition T-a28 using the composition S-a28 having a hydrophilic functional group (a) content of 5.0% by mass or more exhibited better storage stability.

Further, in comparison of the water-based coating compositions T-a24 and T-a30 (Examples 2-24 and 2-30) using the semicarbazide compositions S-a24 and S-a30 (Examples 1-24 and 1-30) having different contents of the hydrophilic functional group (a), the water-based coating composition T-a24 using the composition S-a24 having a hydrophilic functional group (a) content of less than 40.0% by mass had further excellent water resistance of the coating film.

Further, in comparison of the water-based coating compositions T-a26 and T-a28 (Examples 2-26 and 2-28) using the semicarbazide compositions S-a26 and S-a28 (Examples 1-26 and 1-28) with different contents of the aliphatic isocyanate, the water-based coating composition T-a26 using the semicarbazide composition S-a26 having an aliphatic isocyanate content of 10% by mass or more had further excellent elongation of the coating film.

Further, in comparison of the water-based coating compositions T-a1 and T-a5 to T-a8 (Examples 2-1 and 2-5 to 2-8) using the semicarbazide compositions S-a1 and S-a5 to S-a8 (Examples 1-1 and 1-5 to 1-8) having different isocyanate skeletons, all of the water-based coating compositions exhibited excellent dispersibility, and the water-based

coating compositions T-a1, T-a7 and T-a8 using the semicarbazide compositions S-a1, S-a7 and S-a8, wherein the isocyanate skeleton further had a biuret skeleton, an allophanate skeleton or an adduct skeleton, had particularly excellent storage stability.

On the other hand, the water-based coating composition T-b1 (Comparative Example 2-1) using the semicarbazide composition S-b1 (Comparative Example 1-1) which did not contain the structure (b) and had a molar ratio (b)/(a) of the structure (b) to the hydrophilic functional group (a) of 0, storage stability and elongation of the coating film were good, but the water resistance of the coating film was poor.

Further, in the water-based coating composition T-b2 (Comparative Example 2-2) using the semicarbazide composition S-b2 (Comparative Example 1-2) having a molar ratio (b)/(a) of more than 10.0, water resistance and elongation when formed into a coating film were good, but storage stability of the coating composition was poor.

Further, in the water-based coating composition T-b3 (Comparative Example 2-3) using the semicarbazide composition S-c1 (Synthesis Example 2-1) which did not have a hydrophilic functional group (a), storage stability and water resistance when formed into a film were good, but elongation when formed into coating a film was poor.

[Industrial applicability]

According to the semicarbazide composition of the present embodiment, it is possible to provide a semicarbazide composition that has excellent storage stability when formed into a water-based coating composition and excellent water resistance and elongation when formed into a coating film. The water-based coating composition of the present embodiment contains the semicarbazide composition and has excellent water resistance and elongation when formed into a coating film.

## Claims

1. A semicarbazide composition comprising a semicarbazide compound (S) derived from an isocyanate compound (N) and a hydrazine, wherein

   the semicarbazide compound (S) has a hydrophilic functional group (a) and a structure (b) represented by the following formula (1), and
   a molar ratio (b)/(a) of the structure (b) to the hydrophilic functional group (a) is 0.1 or more and 10.0 or less.

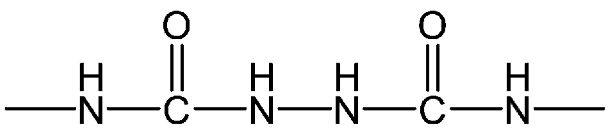

(I)

2. The semicarbazide composition according to Claim 1, wherein the semicarbazide compound (S) comprises a semicarbazide compound derived from a hydrazine and at least one isocyanate compound (N) selected from the group consisting of an aliphatic isocyanate and an alicyclic isocyanate.

3. The semicarbazide composition according to Claim 1 or 2, wherein

   the semicarbazide compound (S) comprises a semicarbazide compound derived from an aliphatic isocyanate and a hydrazine, and
   a content of the aliphatic isocyanate is 10% by mass or more and 99% by mass or less with respect to a total mass of the isocyanate compound (N).

4. The semicarbazide composition according to any one of Claims 1 to 3, wherein

   the isocyanate compound (N) comprises an aliphatic isocyanate and an alicyclic isocyanate, and
   a content of the aliphatic isocyanate is 10% by mass or more and 99% by mass or less with respect to a total mass of the isocyanate compound (N).

5. The semicarbazide composition according to any one of Claims 1 to 4, wherein the isocyanate compound (N) comprises at least one compound selected from the group consisting of a butane diisocyanate, a pentamethylene diisocyanate, a hexamethylene diisocyanate, a trimethylhexamethylene diisocyanate, a 4-isocyanatomethyl-1,8-octamethylene diisocyanate, a lysine ester triisocyanate and a polyisocyanate

derived therefrom.

6. The semicarbazide composition according to any one of Claims 1 to 5, wherein
the hydrophilic functional group (a) is one or more functional groups selected from the group consisting of a nonionic hydrophilic functional group and an anionic hydrophilic functional group.

7. The semicarbazide composition according to any one of Claims 1 to 6, wherein a content of the hydrophilic functional group (a) is 5% by mass or more and 40% by mass or less with respect to a total mass of the semicarbazide compound (S).

8. The semicarbazide composition according to any one of Claims 1 to 7, further comprising a hydrophilic organic solvent (H).

9. The semicarbazide composition according to Claim 8, wherein a content of the hydrophilic organic solvent (H) is 0.5% by mass or more and 50% by mass or less with respect to a total mass of the semicarbazide composition.

10. A water-based coating composition, comprising the semicarbazide composition according to any one of Claims 1 to 9 and a resin (C) having a carbonyl group.

**INTERNATIONAL SEARCH REPORT**

International application No. **PCT/JP2021/040965**

**A. CLASSIFICATION OF SUBJECT MATTER**

***C07C 281/06***(2006.01)i; ***C09D 175/12***(2006.01)i; ***C08G 18/32***(2006.01)i; ***C09D 7/65***(2018.01)i
FI: C08G18/32 028; C09D175/12; C09D7/65; C07C281/06

According to International Patent Classification (IPC) or to both national classification and IPC

**B. FIELDS SEARCHED**

Minimum documentation searched (classification system followed by classification symbols)

C07C281/06; C09D; C08G18/00-18/87

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 54-26897 A (BAYER AG) 28 February 1979 (1979-02-28) claims, p. 3, upper right column, line 5 to lower left column, line 5, p. 3, lower right column, line 20 to p. 4, upper right column, line 16, p. 6, upper right column, line 16 to p. 7, upper left column, line 7, p. 9, line 14 to p. 10, upper left column, line 2, p. 10, lines 9-15, examples 1, 5 | 1-9 |
| Y | | 10 |
| X | JP 2003-252847 A (ASAHI KASEI CORP) 10 September 2003 (2003-09-10) claims, paragraphs [0006], [0059], [0060] (formulas 49, 50) paragraphs [0079], [0084] | 1, 2, 6-10 |
| Y | | 10 |
| A | | 3-5 |
| X | JP 2001-3004 A (ASAHI CHEM IND CO LTD) 09 January 2001 (2001-01-09) claims, paragraphs [0017], [0041], examples | 1-10 |
| Y | | 10 |
| Y | JP 10-231281 A (ASAHI CHEM IND CO LTD) 02 September 1998 (1998-09-02) claims | 10 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **06 January 2022** | **18 January 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/040965**

| Patent document cited in search report | Publication date (day/month/year) | Patent family member(s) | Publication date (day/month/year) |
|---|---|---|---|
| JP 54-26897 A | 28 February 1979 | US 4240942 A claims, column 4, lines 31-59, column 7, line 19 to column 8, line 3, column 11, line 55 to column 12, line 18, examples 1, 5 | |
| JP 2003-252847 A | 10 September 2003 | (Family: none) | |
| JP 2001-3004 A | 09 January 2001 | (Family: none) | |
| JP 10-231281 A | 02 September 1998 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 3073201 B
- JP 3521991 B
- JP 5990277 B
- JP 2005042023 A
- WO 202011100 A
- JP 508 T